# EUROPÄISCHE PATENTANMELDUNG

(11) **EP 4 328 218 A2**
(43) Veröffentlichungstag der Anmeldung: **28.02.2024**
(21) Anmeldenummer: 24150976.9
(22) Anmeldetag: 28.02.2013
(51) Int. Cl.: C07D 239/84

(54) **PHARMAZEUTISCHE ZUBEREITUNG ENTHALTEND EIN ANTIVIRAL WIRKSAMES DIHYDROCHINAZOLINDERIVAT**

(30) Priorität: 29.02.2012 DE 102012101680
(62) Teilanmeldung aus: 19176985.0
(71) Anmelder: AIC246 AG & Co. KG, 42117 Wuppertal (DE)
(72) Erfinder: PAULUS, Kerstin, 40882 Ratingen (DE); SCHWAB, Wilfried, 14542 Werder (Havel) (DE); GRUNDER, Dominique, 3123 Belp (CH); VAN HOOGEVEST, Peter, 67433 Neustadt an der Weinstraße (DE)
(74) Vertreter: Kilger, Ute

(57) **Zusammenfassung**

Die Erfindung betrifft pharmazeutische Zubereitungen, insbesondere zur intravenösen Verabreichung, die {8-Fluor-2-[4-(3-methoxyphenyl)piperazin-1-yl]-3-[2-methoxy-5-(trifluormethyl)phenyl]-3,4-dihydrochinazolin-4-yl}essigsäure in Kombination mit zumindest einem Hilfsstoff ausgewählt aus den Cyclodextrinen, Lysin und Arginin enthalten, Verfahren zu ihrer Herstellung sowie ihre Verwendung zur Behandlung und/oder Prophylaxe von Krankheiten, insbesondere ihre Verwendung als antivirales Mittel vorzugsweise gegen Cytomegalieviren.

## Beschreibung

Die vorliegende Erfindung betrifft eine pharmazeutische Zubereitung, die {8-Fluor-2-[4-(3-methoxyphenyl)piperazin-i-yl]-3-[2-methoxy-5-(trifluormethyl)phenyl]-3,4-dihydrochinazolin-4-yl}essigsäure oder ein Salz, ein Solvat oder ein Solvat eines Salzes davon enthält.

Die Erfindung betrifft ferner Verfahren zu deren Herstellung, deren Verwendung zur Behandlung und/oder Prophylaxe von Virusinfektionen sowie deren Verwendung zur Herstellung von Arzneimitteln zur Behandlung und/oder Prophylaxe von Virusinfektionen, insbesondere zur Behandlung von Infektionen mit dem humanen Cytomegalievirus (HCMV) oder einem anderen Vertreter der Gruppe der Herpes viridae.

{8-Fluor-2-[4-(3-methoxyphenyl)piperazin-1-yl]-3-[2-methoxy-5-(trifluormethyl)phenyl]-3,4-dihydrochinazolin-4-yl}essigsäure ist zum Beispiel aus der WO 2004/096778 bekannt, deren Offenbarung durch Bezugnahme vollumfänglich hierin aufgenommen ist, und wurde von der Anmelderin als aussichtsreicher Kandidat für eine antiviral wirksame Substanz, insbesondere zur Bekämpfung von Infektionen mit dem humanen Cytomegalievirus, entwickelt. Bei der Entwicklung hat sich allerdings herausgestellt, dass Probleme mit der Löslichkeit der Substanz auftraten und insbesondere hat es sich als kompliziert erwiesen, stabile Formulierungen zur intravenösen Verabreichung oder feste gut lösliche Zubereitungen zur Herstellung von Lösungen zur intravenösen Verabreichungen herzustellen.

Es ist somit eine Aufgabe der Erfindung, eine pharmazeutische Zubereitung insbesondere zur intravenösen Verabreichung zu beschreiben, die {8-Fluor-2-[4-(3-methoxyphenyl)piperazin-i-yl]-3-[2-methoxy-5-(trifluormethyl)phenyl]-3,4-di-hydrochinazolin-4-yl}essigsäure enthält, die über lange Zeit stabil und lagerfähig ist, und die ferner einen im Wesentlichen physiologischen pH aufweist.

Es ist eine weitere Aufgabe der Erfindung, eine pharmazeutische Zubereitung zu beschreiben, mit der auf einfache und zuverlässige Weise pharmazeutische Zubereitungen zur intravenösen Verabreichung hergestellt werden können, die {8-Fluor-2-[4-(3-methoxyphenyl)piperazin-1-yl]-3-[2-methoxy-5-(trifluormethyl)phenyl]-3,4-dihydro-chinazolin-4-yl}essigsäure enthalten, die dann ebenfalls über eine ausreichende Zeit, z.B. von mehr als 24 Stunden, stabil sind.

Unter "Stabilität" wird im Rahmen der Erfindung sowohl die chemische Stabilität der Inhaltsstoffe der pharmazeutischen Zubereitung als auch die Stabilität der Lösung selbst verstanden. Insbesondere soll die erfindungsgemäße Zubereitung gegenüber einem Präzipitieren der Inhaltsstoffe stabil sein.

In dem Kontext bedeutet der Begriff "Stabilität" für die erfindungsgemäßen flüssigen pharmazeutischen Zubereitungen bei 2°C bis 8°C, oder bei 25°C, oder bei 40°C, ein Mindestanteil an {8-Fluor-2-[4-(3-methoxyphenyl)piperazin-1-yl]-3-[2-methoxy-5-(trifluormethyl)phenyl]-3,4-dihydrochinazolin-4-yl}essigsäure von > 90%, bevorzugt > 95% für mindestens zwei, bevorzugt mindestens drei, am meisten bevorzugt für mindestens sechs Wochen Lagerung, wenn die besagten flüssigen pharmazeutischen Zubereitungen mittels einer der HPLC-Methoden 1-3 gemessen werden. Diese besagte Stabilität der flüssigen pharmazeutischen Zubereitungen wird im Rahmen der Erfindung als ausreichend angesehen.

Ferner bezeichnet der Begriff "Stabilität" für die erfindungsgemäßen pharmazeutischen Zubereitungen nach Verdünnen oder Rekonstituieren auf eine Endkonzentration von 0,8 - 10 mg/ml zur Infusion bei 2°C bis 8°C, einen Mindestanteil an {8-Fluor-2-[4-(3-methoxyphenyl)piperazin-1-yl]-3-[2-methoxy-5-(trifluormethyl)phenyl]-3,4-dihydro-chinazolin-4-yl}essigsäure von > 90%, bevorzugt > 95% für mindestens vier Stunden, vorzugsweise mindestens sechs Stunden, am meisten bevorzugt mindestens 24 Stunden Lagerung, wenn die besagten flüssigen pharmazeutischen Zubereitungen nach Verdünnen oder Rekonstituieren mittels einer der HPLC-Methoden 1 - 3 gemessen werden. Diese besagte Stabilität der pharmazeutischen Zubereitungen nach Verdünnen oder Rekonstituieren wird im Rahmen der Erfindung als ausreichend angesehen.

Überraschenderweise wurde herausgefunden, dass pharmazeutische Zubereitungen insbesondere zur intravenösen Verabreichung, die {8-Fluor-2-[4-(3-methoxyphenyl)piperazin-1-yl]-3-[2-methoxy-5-(trifluormethyl)phenyl]-3,4-dihydro-chinazolin-4-yl}essigsäure sowie Wasser enthalten, dadurch stabilisiert werden können, dass zumindest ein Hilfsstoff zugegeben wird, der ausgewählt ist aus den Cyclodextrinen, Lysin und Arginin. Ferner wurde herausgefunden, dass solche Zubereitungen lyophilisiert werden können, um eine stabile feste pharmazeutische Zubereitung zu erhalten, die auf einfache Weise z.B. durch Zugabe von Wasser für Injektionszwecke rekonstituiert werden kann, wodurch wiederum eine stabile pharmazeutische Zubereitung, z.B. zur intravenöse Verabreichung, erhalten werden kann.

Gegenstand der Erfindung sind somit pharmazeutische Zubereitungen insbesondere zur intravenösen Verabreichung, die Folgendes aufweisen, nämlich:
a) {8-Fluor-2-[4-(3-methoxyphenyl)piperazin-1-yl]-3-[2-methoxy-5-(trifluormethyl)phenyl]-3,4-dihydrochinazolin-4-yl}essigsäure oder ein Salz, ein Solvat oder ein Solvat eines Salzes davon,
b) zumindest einen Hilfsstoff ausgewählt aus den Cyclodextrinen, Lysin und Arginin, und
c) Wasser.

Gegenstand der Erfindung sind ferner pharmazeutische Zubereitungen, die durch Lyophilisieren der oben genannten pharmazeutischen Zubereitung hergestellt werden.

Unter Salzen werden im Rahmen der Erfindung physiologisch unbedenkliche Salze von {8-Fluor-2-[4-(3-methoxyphenyl)piperazin-1-yl]-3-[2-methoxy-5-(trifluormethyl)phenyl]-3,4-dihydrochinazolin-4-yl}essigsäure verstanden. Physiologisch unbedenkliche Salze von {8-Fluor-2-[4-(3-methoxyphenyl)piperazin-1-yl]-3-[2-methoxy-5-(trifluormethyl)phenyl]-3,4-dihydrochinazolin-4-yl}essigsäure umfassen Säureadditionssalze von Mineralsäuren, Carbonsäuren und Sulfonsäuren, zum Beispiel von Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Citronensäure, Fumarsäure, Maleinsäure und Benzoesäure.

Physiologisch unbedenkliche Salze von {8-Fluor-2-[4-(3-methoxyphenyl)piperazin-1-yl]-3-[2-methoxy-5-(trifluormethyl)phenyl]-3,4-dihydrochinazolin-4-yl}essigsäure umfassen auch Salze üblicher Basen, wie beispielhaft und vorzugsweise Alkalimetallsalze (z.B. Natrium- und Kaliumsalze), Erdalkalimetallsalze (z.B. Calcium- und Magnesiumsalze), Ammoniumsalze abgeleitet von Ammoniak oder organischen Aminen mit 1 bis 16 C-Atomen, wie beispielhaft und vorzugsweise Monoethylamin, Diethylamin, Triethylamin, Ethyldiisopropylamin, Monoethanolamin, Diethanolamin, Triethanolamin, Dicyclohexylamin, Dimethylaminoethanol, 2-Amino-2-methyl-1,3-propandiol, Procain, Dibenzylamin, N-Methylmorpholin, Ethylendiamin und N-Methylpiperidin, sowie die Salze basischer Aminosäuren.

Als Solvate werden im Rahmen der Erfindung solche Formen der {8-Fluor-2-[4-(3-methoxyphenyl)piperazin-1-yl]-3-[2-methoxy-5-(trifluormethyl)phenyl]-3,4-dihydrochinazolin-4-yl}essigsäure bezeichnet, welche durch Koordination mit Lösungsmittelmolekülen einen Komplex bilden. Hydrate sind eine spezielle Form der Solvate, bei denen die Koordination mit Wasser erfolgt.

Wie es einem Fachmann ohne Weiteres ersichtlich ist, weist {8-Fluor-2-[4-(3-methoxyphenyl)piperazin-1-yl]-3-[2-methoxy-5-(trifluormethyl)phenyl]-3,4-dihydrochinazolin-4-yl}essigsäure am Kohlenstoff in 4-Position des Dihydrochinazolenrings ein Stereozentrum auf. Im Rahmen der vorliegenden Erfindung ist besonders bevorzugt, wenn dieser Kohlenstoff die S-Konfiguration aufweist.

Unter einem Cyclodextrin gemäß der Erfindung wird jegliches modifizierte oder nicht modifizierte Cyclodextrin verstanden. Bevorzugt sind hierbei aufgrund der Größe der Kavität im Ring β-Cyclodextrine und insbesondere modifizierte β-Cyclodextrine wie beispielsweise Hydroxyalkyl-β-Cyclodextrine z.B. Hydroxymethyl-β-Cyclodextrin, Hydroxyethyl-β-Cyclodextrin oder Hydroxypropyl-β-Cyclodextrin, Alkyl-Hydroxyalkyl-β-Cyclodextrine z.B. Methyl-Hydroxypropyl-β-Cyclodextrine oder Ethyl-Hydroxypropyl-Cyclodextrine oder Sulfoalkyl-Cyclodextrine.

Das im Rahmen der Erfindung in der Zubereitung verwendete Wasser ist üblicherweise Wasser für Injektionszwecke.

Der Ausdruck "aufweisen" bzw. "aufweisend" bezeichnet im Rahmen der vorliegenden Erfindung eine offene Aufzählung und schließt neben den ausdrücklich genannten Bestandteilen bzw. Schritten andere Bestandteile bzw. Schritte nicht aus.

Der Ausdruck "bestehen aus" bzw. "bestehend aus" bezeichnet im Rahmen der vorliegenden Erfindung eine geschlossene Aufzählung und schließt neben den ausdrücklich genannten Bestandteilen bzw. Schritten jegliche andere Bestandteile bzw. Schritte aus.

Der Ausdruck "im Wesentlichen bestehen aus" bzw. "im Wesentlichen bestehend aus" bezeichnet im Rahmen der vorliegenden Erfindung eine teilweise geschlossene Aufzählung und bezeichnet Verfahren bzw. Zubereitungen die neben den genannten Bestandteilen bzw. Schritten nur noch solche weiteren Bestandteile bzw. Schritte aufweisen, die den erfindungsgemäßen Charakter der Zubereitung bzw. des Verfahrens nicht materiell verändern oder die in Mengen vorliegen, die den erfindungsgemäßen Charakter der Zubereitung bzw. des Verfahrens nicht materiell verändern.

Wenn im Rahmen der vorliegenden Erfindung eine Zubereitung oder ein Verfahren unter Verwendung des Ausdrucks "aufweisen" bzw. "aufweisend" beschrieben ist, schließt dies ausdrücklich Zubereitungen oder Verfahren ein die aus den genannten Bestandteilen bzw. Schritten bestehen oder im Wesentlichen aus den genannten Bestandteilen bzw. Schritten bestehen.

Es ist im Rahmen der Erfindung bevorzugt, wenn die erfindungsgemäße pharmazeutische Zubereitung ferner zumindest einen Puffer aufweist, der vorzugsweise ausgewählt ist aus den Phosphat-Puffern, den Tris-Puffern und den Citrat-Puffern.

Durch die Zugabe des Puffers kann insbesondere sichergestellt werden, dass die Zubereitung stets einen physiologischen pH aufweist, wobei die genannten Puffer insbesondere aufgrund ihrer guten Verträglichkeit bevorzugt sind.

Es ist ferner im Rahmen der Erfindung bevorzugt, wenn die pharmazeutische Zubereitung gemäß der Erfindung ferner zumindest einen Zucker aufweist, der vorzugsweise ausgewählt ist aus der Gruppe bestehend aus Glucose, Saccharose, Lactose, Maltose, Trehalose, Sorbit und Mannit.

Es hat sich gezeigt, dass die pharmazeutische Zubereitung gemäß der Erfindung durch die Zugabe eines Zuckers und insbesondere der oben explizit genannten Zucker noch einmal deutlich stabilisiert werden kann. Ferner hat sich gezeigt, dass sich durch die Zugabe eines Zuckers die Herstellung einer festen Zubereitung durch Lyophilisieren und ein erneutes Rekonstituieren einer solchen festen Zubereitung zur Herstellung einer pharmazeutischen Zubereitung insbesondere zur intravenösen Verabreichung erleichtert werden kann. Ferner dient die Zugabe des zumindest einen Zucker dazu die Osmolalität der Lösung einzustellen und eine ggf. auftretende Hämolyse zu unterdrücken.

Es ist ferner im Rahmen der Erfindung bevorzugt, wenn {8-Fluor-2-[4-(3-methoxyphenyl)piperazin-1-yl]-3-[2-methoxy-5-(trifluormethyl)phenyl]-3,4-di-hydrochinazolin-4-yl}essigsäure oder ein Salz, ein Solvat oder ein Solvat eines Salzes davon in einer Menge in der pharmazeutischen Zubereitung vorliegt, die 1 bis 100 mg, vorzugsweise 2 bis 50 mg, noch bevorzugter 2 bis 25 mg und insbesondere 5 bis 20 mg reiner Wirkstoff pro ml Zubereitung entspricht.

Ein Gehalt an {8-Fluor-2-[4-(3-methoxyphenyl)piperazin-1-yl]-3-[2-methoxy-5-(trifluormethyl)phenyl]-3,4-dihydrochinazolin-4-yl}essigsäure in den oben genannten Bereichen hat sich im Hinblick auf die Stabilität der Lösung, sowie im Hinblick auf eine einfache Lagerung als vorteilhaft erwiesen.

Es ist ferner im Rahmen der Erfindung bevorzugt, wenn die Zubereitung einen pH von 7,5 bis 8,5 aufweist.

Der oben genannte pH-Bereich hat sich aufgrund dessen, dass es sich hierbei um einen pH im Bereich eines physiologischen pHs handelt, als vorteilhaft erwiesen. Ferner hat sich gezeigt, dass die Löslichkeit der erfindungsgemäßen pharmazeutischen Zubereitung im leicht alkalischen Bereich, d.h. in einem Bereich größer als 7,0, noch einmal deutlich besser ist als bei einem pH-Wert von 7,0 oder weniger.

Es ist ferner im Rahmen der Erfindung bevorzugt, wenn der zumindest eine Hilfsstoff in einer Menge von 1 bis 5 Äquivalenten, vorzugsweise von 2 bis 5 Äquivalenten und besonders bevorzugt von 2,5 bis 4,5 Äquivalenten bezogen auf den Gehalt an {8-Fluor-2-[4-(3-methoxyphenyl)piperazin-1-yl]-3-[2-methoxy-5-(trifluormethyl)phenyl]-3,4-dihydrochinazolin-4-yl}essigsäure in der pharmazeutischen Zubereitung vorliegt.

Es ist ferner im Rahmen der Erfindung bevorzugt, wenn die Zubereitung bezogen auf den Gehalt an {8-Fluor-2-[4-(3-methoxyphenyl)piperazin-1-yl]-3-[2-methoxy-5-(trifluormethyl)phenyl]-3,4-dihydrochinazolin-4-yl}essigsäure 1 bis 10 Äquivalente, vorzugsweise 2 bis 7 Äquivalente und insbesondere 2,5 bis 5 Äquivalente Cyclodextrin sowie 0 bis 2,0 Äquivalente, vorzugsweise 0,5 bis 1,5 Äquivalente und insbesondere 0,75 bis 0,9 Äquivalente NaOH aufweist.

Unter Äquivalenten im Rahmen der Erfindung werden molare Äquivalente verstanden. Es hat sich gezeigt, dass die Zugabe von weniger Hilfsstoff als in den oben genannten Bereichen als Untergrenze genannt, zu einer nicht ausreichenden Stabilisierung der Lösung führt. Eine Zugabe von Mengen an Hilfsstoff, die größer sind als die genannten oberen Grenzen erbringt keine weiteren Vorteile im Hinblick auf die Stabilität der Zubereitung. Es wird ferner befürchtet, dass eine Zugabe größerer Mengen an Hilfsstoff ferner zu Interaktionen mit dem aktiven Wirkstoff und somit eher zu einer Verminderung der Wirksamkeit der Zubereitung führen wird.

Besonders bevorzugt im Rahmen der Erfindung ist eine pharmazeutische Zubereitung, die bezogen auf 100 ml Zubereitung Folgendes aufweist:
a) 0,25 - 2,0 g, vorzugsweise 0,5 - 1,25 g {8-Fluor-2-[4-(3-methoxyphenyl)piperazin-1-yl]-3-[2-methoxy-5-(trifluormethyl)phenyl]-3,4-dihydrochinazolin-4-yl}essigsäure oder ein Salz, ein Solvat oder ein Solvat eines Salzes davon,
b) 0,25 - 2,5 g, vorzugsweise 0,5 g - 1,5 g Arginin,
c) 1,5 - 9,5 g, vorzugsweise 2,0 - 4,75 g Glucose,
d) 0,5 - 4,0 g, vorzugsweise 0,75 - 2,0 g NaH₂PO₄, und
e) Wasser,
wobei die Zubereitung einen pH von 7,5 bis 8,5, vorzugsweise 7,7 bis 8,0 aufweist.

Ferner ist im Rahmen der Erfindung ist eine pharmazeutische Zubereitung besonders bevorzugt, die bezogen auf 100 ml Zubereitung Folgendes aufweist:
a) 0,5 - 2,5 g, vorzugsweise 1,0 - 2,0 g {8-Fluor-2-[4-(3-methoxyphenyl)piperazin-1-yl]-3-[2-methoxy-5-(trifluormethyl)phenyl]-3,4-dihydrochinazolin-4-yl}essigsäure oder ein Salz, ein Solvat oder ein Solvat eines Salzes davon,
b) 10,0 - 30,0 g, vorzugsweise 12,5 g - 22,5 g HP-β-Cyclodextrin,
c) 0,0 -350 mg, vorzugsweise 75 - 225 mg, insbesondere 100 - 125 mg NaOH, und
d) Wasser,
wobei die Zubereitung einen pH von 7,5 bis 8,5 aufweist.

In letzterer Zubereitung wird das NaOH bevorzugt in Form einer ca. 0,1 M wässrigen Lösung eingesetzt.

Es hat sich gezeigt, dass pharmazeutische Zubereitungen mit diesen Zusammensetzungen sowohl im Hinblick auf die klinische Effektivität als auch die Stabilität besonders vorteilhaft sind.

Die pharmazeutischen Zubereitungen gemäß der Erfindung werden im Allgemeinen hergestellt, indem zuerst eine wässrige Lösung des Hilfsstoffs hergestellt wird und dann {8-Fluor-2-[4-(3-methoxyphenyl)piperazin-1-yl]-3-[2-methoxy-5-(trifluormethyl)phenyl]-3,4-dihydrochinazolin-4-yl}essigsäure zu dieser Lösung zugegeben wird, ggf. gefolgt von einer Zugabe anderer Zusatzstoffe wie beispielsweise des zumindest einen Zuckers und/oder des zumindest einen Puffers. Nach Zugabe sämtlicher Inhaltstoffe wird dann der pH-Wert der pharmazeutischen Zubereitung auf den gewünschten Wert eingestellt, wobei insbesondere darauf zu achten ist, dass, wenn der pH-Wert durch Zugabe einer Säure oder eines Puffers von einem im Alkalischen liegenden Wert in Richtung des physiologischen pH-Werts eingestellt wird, diese Einstellung langsam und vorsichtig erfolgt, um ein Ausfallen von {8-Fluor-2-[4-(3-methoxyphenyl)piperazin-1-yl]-3-[2-methoxy-5-(trifluormethyl)phenyl]-3,4-dihydrochinazolin-4-yl}essigsäure durch eine starke lokale Erniedrigung des pH-Werts zu vermeiden.

Es ist ferner möglich, zuerst einzelne Lösungen herzustellen, wobei eine Lösung den Hilfsstoff sowie {8-Fluor-2-[4-(3-methoxyphenyl)piperazin-1-yl]-3-[2-methoxy-5-(trifluormethyl)phenyl]-3,4-dihydrochinazolin-4-yl}essigsäure enthält, und die andere Lösung die weiteren Hilfsstoffe wie beispielsweise den zumindest einen Zucker und/oder den zumindest einen Puffer enthält, wobei im nächsten Schritt die Lösungen auf den gewünschten pH eingestellt und dann miteinander gemischt werden.

Es ist ferner möglich die {8-Fluor-2-[4-(3-methoxyphenyl)piperazin-1-yl]-3-[2-methoxy-5-(trifluormethyl)phenyl]-3,4-dihydrochinazolin-4-yl}essigsäure in einer wässrigen basischen Lösung z.B. einer Lösung eines Alkalimetallhydroxids vorzugsweise einer NaOH-Lösung zumindest teilweise zu lösen, dann den Hilfsstoff, sowie ggf. die anderen Inhaltsstoffe zu der Lösung zuzugeben und ggf. die Lösung auf den gewünschten pH-Wert einzustellen.

Es ist ferner möglich, die Lösungen, die nach den oben genannten Verfahren erhalten werden, zu lyophilisieren, um die erfindungsgemäßen festen pharmazeutischen Zubereitungen zu erhalten.

Gegenstand der Erfindung ist somit auch ein Verfahren zum Herstellen einer erfindungsgemäßen pharmazeutischen Zubereitung mit den folgenden Schritten:
A) Lösen des zumindest einen Hilfsstoffs in dem Wasser,
B) Zugeben von {8-Fluor-2-[4-(3-methoxyphenyl)piperazin-1-yl]-3-[2-methoxy-5-(trifluormethyl)phenyl]-3,4-dihydrochinazolin-4-yl}essigsäure oder einem Salz, einem Solvat oder einem Solvat eines Salzes davon zu der in Schritt A) erhaltenen Lösung,
C) ggf. Zugeben des zumindest einen Zuckers und/oder des zumindest einen Puffers,
D) Einstellen des pHs auf den gewünschten Wert um eine pharmazeutische Zubereitung zu erhalten, und
E) Sterilfiltrieren der in Schritt D) erhaltenen Lösung und Abfüllen in geeignete Behälter.
F) ggf. Endsterilisieren der in Schritt E) erhaltenen Lösung durch Erhitzen.

Gegenstand der Erfindung ist ferner ein Verfahren zur Herstellung einer erfindungsgemäßen pharmazeutischen Zubereitung mit den folgenden Schritten:
I.) Lösen des zumindest einen Hilfsstoffes in einem Teil des Wassers,
II.) Zugeben von {8-Fluor-2-[4-(3-methoxyphenyl)piperazin-1-yl]-3-[2-methoxy-5-(trifluormethyl)phenyl]-3,4-dihydrochinazolin-4-yl}essigsäure oder einem Salz, einem Solvat oder einem Solvat eines Salzes davon zu der in Schritt I.) erhaltenen Lösung,
III.) ggf. Einstellen des pHs der in Schritt II.) erhaltenen Lösung auf den gewünschten Wert um eine erste Lösung zu erhalten,
IV.) Lösen zumindest eines Zuckers und/oder eines Puffers in einem Teil des Wassers,
V.) ggf. Einstellen des pHs der in Schritt IV.) erhaltenen Lösung auf den gewünschten Wert um eine zweite Lösung zu erhalten,
VI.) Vermischen der ersten und der zweiten Lösung um eine pharmazeutische Zubereitung zu erhalten, und
VII.) Sterilfiltrieren der in Schritt VI.) erhaltenen Lösung und Abfüllen in geeignete Behälter.
VIII.)ggf. Endsterilisieren der in Schritt VII.) erhaltenen Lösung durch Erhitzen.

Gegenstand der Erfindung ist ferner ein Verfahren zur Herstellung einer erfindungsgemäßen pharmazeutischen Zubereitung mit den folgenden Schritten:
a.) Zugeben von {8-Fluor-2-[4-(3-methoxyphenyl)piperazin-1-yl]-3-[2-methoxy-5-(trifluormethyl)phenyl]-3,4-dihydrochinazolin-4-yl}essigsäure oder einem Salz, einem Solvat oder einem Solvat eines Salzes zu einer wässrigen NaOH-Lösung, vorzugsweise einer wässrigen 0,1 M NaOH-Lösung zur Herstellung einer Lösung oder Suspension,
b.) Zugeben von Wasser zu der in Schritt a.) erhaltenen Lösung bzw. Suspension,
c.) Zugeben von Cyclodextrin und NaCl zu der in Schritt b.) erhaltenen Lösung bzw. Suspension,
d.) Sterilfiltrieren der in Schritt c.) erhaltenen Lösung und Abfüllen in geeignete Behälter.
e.) ggf. Endsterilisieren der in Schritt d.) erhaltenen Lösung durch Erhitzen.

Gegenstand der Erfindung ist weiter ein Verfahren zur Herstellung einer festen pharmazeutischen Zubereitung, wobei eine nach den oben genannten Verfahren hergestellte pharmazeutische Zubereitung lyophilisiert wird.

Die zur Herstellung der erfindungsgemäßen pharmazeutischen Zubereitung verwendete {8-Fluor-2-[4-(3-methoxyphenyl)piperazin-1-yl]-3-[2-methoxy-5-(trifluormethyl)phenyl]-3,4-dihydrochinazolin-4-yl}essigsäure bzw. deren Salze, Solvate und Solvate der Salze sind bekannt und können zum Beispiel nach dem in der WO 2006/133822 beschriebenen Verfahren hergestellt werden.

Insbesondere erfolgt die Herstellung durch die Verseifung des Esters einer Verbindung der Formel (II) 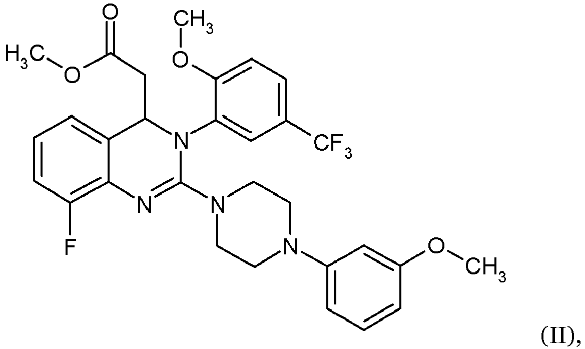 mit einer Base.

Die Verbindung der Formel (II) kann hergestellt werden durch Umsetzung einer Verbindung der Formel (III) 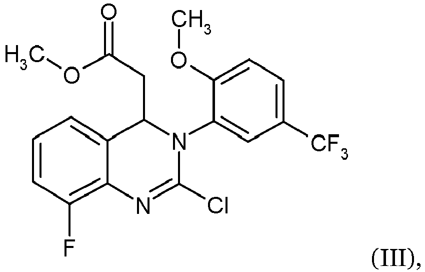 in Gegenwart einer Base mit einer Verbindung der Formel (IV).

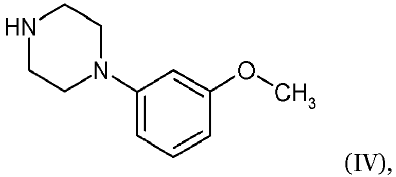

Die Verbindung der Formel (III) kann hergestellt werden durch Umsetzung einer Verbindung der Formel (V) 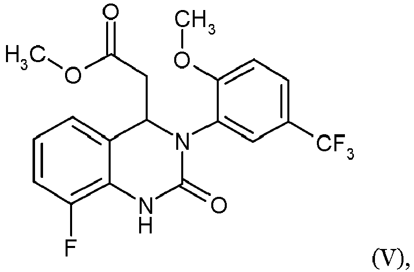 mit Phosphoroxychlorid, Phosphortrichlorid oder Phosphorpentachlorid in Gegenwart einer Base.

Die Verbindung der Formel (V) kann hergestellt werden durch Umsetzung einer Verbindung der Formel (VI) 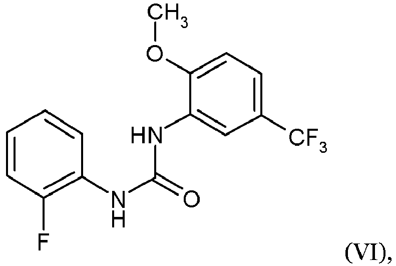 in der ersten Stufe mit Acrylsäuremethylester in Gegenwart eines Palladiumkatalysators und Oleum und in der zweiten Stufe mit einer Base.

Verbindungen der Formeln (IV) und (VI) sind dem Fachmann an sich bekannt oder lassen sich nach üblichen, literaturbekannten Verfahren herstellen.

Die Verseifung des Esters einer Verbindung der Formel (II) zu {8-Fluor-2-[4-(3-methoxyphenyl)piperazin-1-yl]-3-[2-methoxy-5-(trifluormethyl)phenyl]-3,4-dihydrochinazolin-4-yl}essigsäure erfolgt durch Umsetzung einer Verbindung der Formel (II) mit einer Base in einem inerten Lösungsmittel, in einem Temperaturbereich von 18°C bis zum Rückfluss des Lösungsmittels, bevorzugt bei 18 bis 50°C, besonders bevorzugt bei 20 bis 30°C, bei Normaldruck, innerhalb von beispielsweise 0,5 bis 10 Stunden, bevorzugt innerhalb von 1 bis 5 Stunden.

Basen sind beispielsweise Alkalihydroxide wie Natrium-, Lithium- oder Kaliumhydroxid, oder Alkalicarbonate wie Cäsiumcarbonat, Natrium- oder Kaliumcarbonat, oder Alkoholate wie Natrium- oder Kaliummethanolat oder Natrium- oder Kaliumethanolat, wobei die Base ggf. in wässriger Lösung vorliegt.

Inerte Lösungsmittel sind beispielsweise Ether wie 1,2-Dimethoxyethan, Methyl-tert-butylether (MTBE), Dioxan, Tetrahydrofuran, Glykoldimethylether oder Diethylenglykoldimethylether, Alkohole wie Methanol, Ethanol, n-Propanol, iso-Propanol, n-Butanol oder tert.-Butanol, oder Wasser, oder Gemische von Lösungsmitteln.

Bevorzugt ist Natriumhydroxid in Wasser und MTBE.

Die Synthese einer Verbindung der Formel (II) aus einer Verbindung der Formel (III) und einer Verbindung der Formel (IV) in Gegenwart einer Base erfolgt in einem inerten Lösungsmittel, in einem Temperaturbereich von 40°C bis zum Rückfluss des Lösungsmittels, bevorzugt bei Rückfluss des Lösungsmittels, bei Normaldruck, innerhalb von beispielsweise 2 bis 48 Stunden, bevorzugt innerhalb von 4 bis 12 Stunden.

Basen sind beispielsweise Aminbasen wie 1,8-Diazabicyclo[5.4.0]undec-7-en (DBU), 1-(3-Methoxyphenyl)piperazin oder Triethylamin, oder andere Basen wie Kalium-tert.-butylat.

Inerte Lösungsmittel sind beispielsweise Chlorbenzol oder Ether wie 1,2-Dimethoxyethan, Dioxan, Glykoldimethylether oder Diethylenglykoldimethylether.

Bevorzugt ist DBU in Dioxan.

Die Umsetzung einer Verbindung der Formel (V) zu einer Verbindung der Formel (III) erfolgt durch Reaktion einer Verbindung der Formel (V) mit Phosphoroxychlorid, Phosphortrichlorid oder Phosphorpentachlorid, bevorzugt ist Phosphoroxychlorid, in Gegenwart einer Base in einem inerten Lösungsmittel, in einem Temperaturbereich von 40°C bis zum Rückfluss des Lösungsmittels, bevorzugt bei Rückfluss des Lösungsmittels, bei Normaldruck, innerhalb von beispielsweise 1 bis 48 Stunden, bevorzugt innerhalb von 2 bis 12 Stunden.

Basen sind beispielsweise Amine wie 1,8-Diazabicyclo[5.4.0]undec-7-en (DBU), Pyridin oder Triethylamin, oder andere Basen wie Kalium-tert.-butylat.

Inerte Lösungsmittel sind beispielsweise Kohlenwasserstoffe wie Benzol, Xylol, Toluol oder Chlorbenzol.

Bevorzugt ist DBU in Chlorbenzol.

Die Umsetzung einer Verbindung der Formel (VI) zu einer Verbindung der Formel (V) erfolgt in der ersten Stufe durch Reaktion einer Verbindung der Formel (VI) mit Acrylsäuremethylester in Gegenwart eines Palladiumkatalysators und Oleum in einem Lösungsmittel in einem Temperaturbereich von 0°C bis 40°C, bevorzugt bei Raumtemperatur und in der zweiten Stufe durch Reaktion mit einer Base in einem inerten Lösungsmittel, in einem Temperaturbereich von 40°C bis zum Rückfluss des Lösungsmittels, bevorzugt bei Rückfluss des Lösungsmittels, bei Normaldruck, innerhalb von beispielsweise 1 bis 48 Stunden, bevorzugt innerhalb von 2 bis 12 Stunden.

Palladiumkatalysatoren in der ersten Stufe sind beispielsweise Palladium(II)acetat, Bis(triphenylphosphin)palladium(II)chlorid, Tetrakis(triphenylphosphin)palladium(o), Bis(tris(o-tolyl)phosphino)palladium-(II)-chlorid oder ein aus Bis(acetonitril)-dichlorpalladium oder Palladium(II)acetat und einem Liganden, beispielsweise Tris(o-tolyl)phosphin, Triphenylphosphin oder Diphenylphosphinoferrocen, hergestellter Palladiumkatalysator.

Lösungsmittel in der ersten Stufe sind beispielsweise organische Säuren wie Essigsäure oder Propionsäure.

Bevorzugt ist Palladium(II)acetat in Essigsäure.

Basen in der zweiten Stufe sind beispielsweise DBU, Triethylamin oder Diisopropylethylamin.

Inerte Lösungsmittel in der zweiten Stufe sind beispielsweise Ether wie 1,2-Dimethoxyethan, Dioxan, Glykoldimethylether oder Diethylenglykoldimethylether, Kohlenwasserstoffe wie Benzol, Xylol oder Toluol, oder andere Lösungsmittel wie Isobutyronitril, Acetonitril, Aceton, Nitrobenzol, Dimethylformamid, Dimethylacetamid, Dimethylsulfoxid oder N-Methylpyrrolidon.

Bevorzugt ist DBU in Aceton.

Die Herstellung der zur Herstellung der erfindungsgemäßen pharmazeutischen Zubereitung verwendeten {8-Fluor-2-[4-(3-methoxyphenyl)piperazin-1-yl]-3-[2-methoxy-5-(trifluormethyl)phenyl]-3,4-dihydrochinazolin-4-yl}essigsäure ist in dem nachstehenden Syntheseschema 1 exemplarisch näher erläutert. Das Syntheseschema ist hierbei rein beispielhaft und in keiner Weise einschränkend zu verstehen.

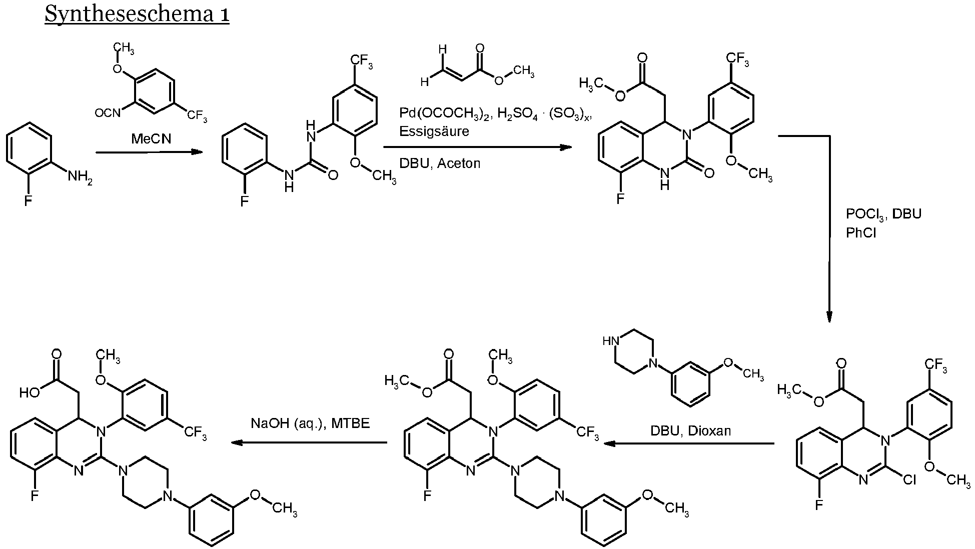

Wie bereits zuvor erwähnt wird die {8-Fluor-2-[4-(3-methoxyphenyl)piperazin-1-yl]-3-[2-methoxy-5-(trifluormethyl)phenyl]-3,4-dihydrochinazolin-4-yl}essigsäure vorzugsweise in Form des S-Enantiomers eingesetzt. Dieses S-Enantiomer kann dabei z.B. wie in dem nachstehenden Syntheseschema 2 dargestellt hergestellt werden.

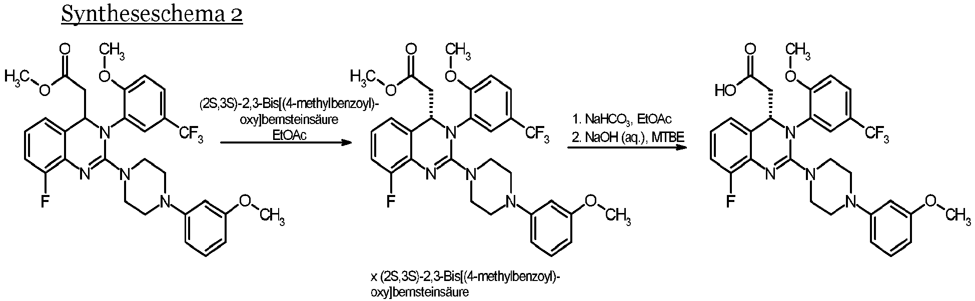

Die in der pharmazeutischen Zubereitung enthaltene {8-Fluor-2-[4-(3-methoxyphenyl)piperazin-1-yl]-3-[2-methoxy-5-(trifluormethyl)phenyl]-3,4-dihydro-chinazolin-4-yl}essigsäure sowie deren Salze, Solvate und Solvate der Salze zeigen eine antivirale Wirkung gegenüber Vertretern der Gruppe der Herpes viridae (Herpesviren), vor allem gegenüber Cytomegalieviren (CMV), insbesondere gegenüber dem humanen Cytomegalievirus (HCMV). Die erfindungsgemäßen pharmazeutischen Zubereitungen sind somit geeignet zur Behandlung und/oder Prophylaxe von Krankheiten, vor allem von Infektionen mit Viren, insbesondere den hierin genannten Viren und den dadurch hervorgerufenen Infektionskrankheiten. Unter einer Virusinfektion wird sowohl eine Infektion mit einem Virus als auch eine durch eine Infektion mit einem Virus hergerufene Krankheit verstanden.

Die erfindungsgemäßen pharmazeutischen Zubereitungen können aufgrund ihrer Eigenschaften zur Herstellung von Arzneimitteln verwendet werden, die zur Prophylaxe und/oder Behandlung von Krankheiten, insbesondere von Virusinfektionen geeignet sind. Als Indikationsgebiet können beispielsweise genannt werden:
1) Behandlung und Prophylaxe von HCMV-Infektionen bei AIDS-Patienten (Retinitis, Pneumonitis, gastrointestinale Infektionen).
2) Behandlung und Prophylaxe von Cytomegalievirus-Infektionen bei Knochenmark- und Organtransplantationspatienten, die an einer HCMV-Pneumonitis, -Enzephalitis, sowie an gastrointestinalen und systemischen HCMV-Infektionen oft lebensbedrohlich erkranken.
3) Behandlung und Prophylaxe von HCMV-Infektionen bei Neugeborenen und Kleinkindern.
4) Behandlung einer akuten HCMV-Infektion bei Schwangeren.
5) Behandlung der HCMV-Infektion bei immunsupprimierten Patienten bei Krebs und Krebs-Therapie.
6) Behandlung von HCMV-positiven Krebspatienten mit dem Ziel, HCMV-vermittelte Tumorprogression zu verringern (vgl. J. Cinatl, et al., FEMS Microbiology Reviews 2004, 28, 59-77).

Bevorzugt werden die erfindungsgemäßen pharmazeutischen Zubereitungen zur Herstellung von Arzneimitteln verwendet, die zur Prophylaxe und/oder Behandlung von Infektionen mit einem Vertreter der Gruppe der Herpes viridae, besonders einem Cytomegalievirus, insbesondere dem humanen Cytomegalievirus, geeignet sind.

Die erfindungsgemäßen pharmazeutischen Zubereitungen können aufgrund ihrer pharmakologischen Eigenschaften allein und bei Bedarf auch in Kombination mit anderen Wirkstoffen, insbesondere antiviralen Wirkstoffen wie beispielsweise Valganciclovir, Ganciclovir, Valacyclovir, Acyclovir, Foscarnet, Cidofovir und verwandten Derivaten zur Behandlung und/oder Prävention von Virusinfektionen, insbesondere von HCMV-Infektionen, eingesetzt werden.

Weiterer Gegenstand der vorliegenden Erfindung ist der Einsatz der erfindungsgemäßen pharmazeutischen Zubereitungen in einem Verfahren zur Behandlung und/oder Prophylaxe von Erkrankungen, vorzugsweise von Virusinfektionen, insbesondere von Infektionen mit dem humanen Cytomegalievirus (HCMV) oder einem anderen Vertreter der Gruppe der Herpes viridae.

Weiterer Gegenstand der vorliegenden Erfindung ist die Verwendung der erfindungsgemäßen pharmazeutischen Zubereitungen zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen pharmazeutischen Zubereitungen zur Herstellung eines Arzneimittels zur Behandlung und/oder Prophylaxe von Erkrankungen, insbesondere der zuvor genannten Erkrankungen.

Weiterer Gegenstand der vorliegenden Erfindung ist ein Verfahren zur Behandlung und/oder Prophylaxe von Erkrankungen, insbesondere der zuvor genannten Erkrankungen, unter Verwendung einer antiviral wirksamen Menge der erfindungsgemäßen pharmazeutischen Zubereitungen.

Der Begriff "antiviral wirksame Menge" bezeichnet die erfindungsgemäßen pharmazeutischen Zubereitungen in einer Darreichungsmenge von mindestens 0,001 mg/kg.

Im Allgemeinen hat es sich als vorteilhaft erwiesen, die pharmazeutischen Zubereitungen so zu verabreichen, dass etwa 0,001 bis 10 mg pro kg, vorzugsweise 0,01 bis 5 mg pro kg Körpergewicht {8-Fluor-2-[4-(3-methoxyphenyl)piperazin-1-yl]-3-[2-methoxy-5-(trifluormethyl)phenyl]-3,4-dihydrochinazolin-4-yl}essigsäure verabreicht werden.

Trotzdem kann es ggf. erforderlich sein, von den genannten Mengen abzuweichen, und zwar in Abhängigkeit vom Körpergewicht, individuellem Verhalten gegenüber dem Wirkstoff und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzeldosen über den Tag zu verteilen.

Die Erfindung wird nun nachstehend anhand von nicht einschränkenden Beispielen näher erläutert.

Die Prozentangaben in den folgenden Tests und Beispielen sind, sofern nicht anders angegeben, Gewichtsprozente, Teile sind Gewichtsanteile, Lösungsmittelverhältnis-, Verdünnungsverhältnis- und Konzentrationsangaben von Flüssiglösungen beziehen sich jeweils auf das Volumen.

In bestimmten Ausführungsformen, betrifft die vorliegende Erfindung die Gegenstände, die in den folgenden Punkten aufgezählt sind:
1. Pharmazeutische Zubereitung, insbesondere zur intravenösen Verabreichung die folgendes aufweist, nämlich:
   a) {8-Fluor-2-[4-(3-methoxyphenyl)piperazin-1-yl]-3-[2-methoxy-5-(trifluormethyl)phenyl]-3,4-dihydrochinazolin-4-yl}essigsäure oder ein Salz, ein Solvat oder ein Solvat eines Salzes davon,
   b) zumindest einen Hilfsstoff ausgewählt aus den Cyclodextrinen, Lysin und Arginin, und
   c) Wasser.
2. Pharmazeutische Zubereitung nach Punkt 1, die ferner zumindest einen Puffer aufweist, der vorzugsweise ausgewählt ist aus den Phosphat-Puffern, den Tris-Puffern und den Citrat-Puffern.
3. Pharmazeutische Zubereitung nach Punkt 1 oder 2, die ferner zumindest einen Zucker aufweist, der vorzugsweise ausgewählt ist aus der Gruppe bestehend aus Glucose, Saccharose, Lactose, Maltose, Trehalose, Sorbit und Mannit.
4. Pharmazeutische Zubereitung nach einem der Punkte 1 bis 3, dadurch gekennzeichnet, dass {8-Fluor-2-[4-(3-methoxyphenyl)piperazin-1-yl]-3-[2-methoxy-5-(trifluormethyl)phenyl]-3,4-dihydrochinazolin-4-yl}essigsäure oder ein Salz, ein Solvat oder ein Solvat eines Salzes davon in einer Menge vorliegt die 1 bis 100 mg, vorzugsweise 2 bis 50 mg, noch bevorzugter 2 bis 25 mg und insbesondere 5 bis 20 mg reiner Wirkstoff pro ml Zubereitung entspricht.
5. Pharmazeutische Zubereitung nach einem der Punkte 1 bis 4, dadurch gekennzeichnet, dass die Zubereitung einen pH von 7,5 bis 8,5 aufweist.
6. Pharmazeutische Zubereitung nach einem der Punkte 1 bis 5, dadurch gekennzeichnet, dass der zumindest eine Hilfsstoff in einer Menge von 1 bis 5 Äquivalenten, vorzugsweise von 2 bis 5 Äquivalenten und besonders bevorzugt von 2,5 bis 4,5 Äquivalenten bezogen auf den Gehalt an {8-Fluor-2-[4-(3-methoxyphenyl)piperazin-1-yl]-3-[2-methoxy-5-(trifluormethyl)phenyl]-3,4-dihydrochinazolin-4-yl}essigsäure vorliegt.
7. Pharmazeutische Zubereitung nach einem der Punkte 1 bis 6, dadurch gekennzeichnet, dass sie bezogen auf 100 ml Folgendes aufweist:
   a) 0,2 - 2,0 g, vorzugsweise 0,5 - 1,25 g {8-Fluor-2-[4-(3-methoxyphenyl)piperazin-1-yl]-3-[2-methoxy-5-(trifluormethyl)phenyl]-3,4-dihydrochinazolin-4-yl}essigsäure oder ein Salz, ein Solvat oder ein Solvat eines Salzes davon,
   b) 0,25 - 2,5 g, vorzugsweise 0,5 - 1,5 g Arginin,
   c) 1,5 - 9,5 g, vorzugsweise 2,0 - 4,75 g Glucose,
   d) 0,6 - 4,0 g, vorzugsweise 0,75 - 2,0 g NaH₂PO₄, und
   e) Wasser,
   wobei die Zubereitung einen pH von 7,5 bis 8,5, vorzugsweise von 7,7 bis 8,0 aufweist.
8. Pharmazeutische Zubereitung nach einem der Punkte 1 bis 5, dadurch gekennzeichnet, dass der Hilfsstoff ausgewählt ist aus den Cyclodextrinen, vorzugsweise den β-Cyclodextrinen und den modifizierten β-Cyclodextrinen insbesondere den Hydroxyalkyl-β-Cyclodextrinen, den Alkyl-Hydroxyalkyl-β-Cyclodextrinen und den Sulfoalkyl-Cyclodextrinen.
9. Pharmazeutische Zubereitung nach Punkt 8, dadurch gekennzeichnet, dass die Zubereitung bezogen auf den Gehalt an {8-Fluor-2-[4-(3-methoxyphenyl)piperazin-1-yl]-3-[2-methoxy-5-(trifluormethyl)phenyl]-3,4-dihydrochinazolin-4-yl}essigsäure 1 bis 10 Äquivalente, vorzugsweise 2 bis 7 Äquivalente und insbesondere 2,5 bis 5 Äquivalente Cyclodextrin sowie 0 bis 2,0 Äquivalente, vorzugsweise 0,5 bis 1,5 Äquivalent und insbesondere 0,75 bis 0,9 Äquivalente NaOH aufweist.
10. Pharmazeutische Zubereitung nach einem der Punkte 1 bis 5 oder nach Punkt 8 oder 9, dadurch gekennzeichnet, dass sie bezogen auf 100 ml Folgendes aufweist:
   a) 0,5 - 2,5 g, vorzugsweise 1,0 - 2,0 g {8-Fluor-2-[4-(3-methoxyphenyl)piperazin-1-yl]-3-[2-methoxy-5-(trifluormethyl)phenyl]-3,4-dihydrochinazolin-4-yl}essigsäure oder ein Salz, ein Solvat oder ein Solvat eines Salzes davon,
   b) 10,0 - 30,0 g, vorzugsweise 12,5 g - 22,5 g HP-β-Cyclodextrin,
   c) 0,0 - 350 mg, vorzugsweise 75 - 225 mg, insbesondere 100 - 125 mg NaOH, und
   d) Wasser,
   wobei die Zubereitung einen pH von 7,5 bis 8,5 aufweist.
11. Feste pharmazeutische Zubereitung, hergestellt durch Lyophilisieren einer pharmazeutischen Zubereitung nach einem der Punkte 1 bis 10.
12. Verfahren zur Herstellung einer pharmazeutischen Zubereitung nach einem der Punkte 1 bis 7, mit den folgenden Schritten, nämlich:
   A) Lösen des zumindest einen Hilfsstoffs in dem Wasser,
   B) Zugeben von {8-Fluor-2-[4-(3-methoxyphenyl)piperazin-1-yl]-3-[2-ethoxy-5-(trifluormethyl)phenyl]-3,4-dihydrochinazolin-4-yl}essigsäure oder einem Salz, einem Solvat oder einem Solvat eines Salzes davon zu der in Schritt A) erhaltenen Lösung,
   C) ggf. Zugeben des zumindest einen Zuckers und/oder des zumindest einen Puffers,
   D) Einstellen des pHs auf den gewünschten Wert, um eine pharmazeutische Zubereitung zu erhalten, und
   E) Sterilfiltrieren der in Schritt D) erhaltenen Lösung und Abfüllen in geeignete Behälter.
   F) ggf. Endsterilisieren der in Schritt E) erhaltenen Lösung durch Erhitzen.
13. Verfahren zur Herstellung einer pharmazeutischen Zubereitung nach einem der Punkte 1 bis 7, mit den folgenden Schritten, nämlich:
   I.) Lösen des zumindest einen Hilfsstoffes in einem Teil des Wassers,
   II.) Zugeben von {8-Fluor-2-[4-(3-methoxyphenyl)piperazin-1-yl]-3-[2-methoxy-5-(trifluormethyl)phenyl]-3,4-dihydrochinazolin-4-yl}essigsäure oder einem Salz, einem Solvat oder einem Solvat eines Salzes davon zu der in Schritt I.) erhaltenen Lösung,
   III.) ggf. Einstellen des pHs der in Schritt II.) erhaltenen Lösung auf den gewünschten Wert, um eine erste Lösung zu erhalten,
   IV.) Lösen zumindest eines Zuckers und/oder eines Puffers in einem Teil des Wassers,
   V.) ggf. Einstellen des pHs der in Schritt IV.) erhaltenen Lösung auf den gewünschten Wert um eine zweite Lösung zu erhalten,
   VI.) Vermischen der ersten und der zweiten Lösung um eine pharmazeutische Zubereitung zu erhalten, und
   VII.) Sterilfiltrieren der in Schritt VI.) erhaltenen Lösung und Abfüllen in geeignete Behälter.
   VIII.) ggf. Endsterilisieren der in Schritt VII.) erhaltenen Lösung durch Erhitzen.
14. Verfahren zur Herstellung einer pharmazeutischen Zubereitung nach einem der Punkte 1 bis 5 oder 8 bis 10, mit den folgenden Schritten, nämlich:
   a.) Zugeben von {8-Fluor-2-[4-(3-methoxyphenyl)piperazin-1-yl]-3-[2-methoxy-5-(trifluormethyl)phenyl]-3,4-dihydrochinazolin-4-yl}essigsäure oder einem Salz, einem Solvat oder einem Solvat eines Salzes zu einer wässrigen NaOH-Lösung, vorzugsweise einer wässrigen 0,1 M NaOH-Lösung, zur Herstellung einer Lösung oder Suspension,
   b.) Zugeben von Wasser zu der in Schritt a.) erhaltenen Lösung bzw. Suspension,
   c.) Zugeben von Cyclodextrin und NaCl zu der in Schritt b.) erhaltenen Lösung bzw. Suspension,
   d.) Sterilfiltrieren der in Schritt c.) erhaltenen Lösung und Abfüllen in geeignete Behälter.
   e.) ggf. Endsterilisieren der in Schritt d.) erhaltenen Lösung durch Erhitzen.
15. Verfahren zur Herstellung einer festen pharmazeutischen Zubereitung nach Punkt 11, umfassend die Herstellung einer pharmazeutischen Zubereitung gemäß einem Verfahren nach einem der Punkte 12 bis 14 gefolgt von einem Schritt des Lyophilisierens der erhaltenen pharmazeutischen Zubereitung, um eine feste pharmazeutische Zubereitung zu erhalten.
16. Pharmazeutische Zubereitung nach einem der Punkte 1 bis 11 zur Verwendung in einem Verfahren zur Behandlung und/oder Prophylaxe von Krankheiten, insbesondere von Virusinfektionen, vorzugsweise von Infektionen mit HCMV oder einem anderen Vertreter der Gruppe der Herpes viridae.
17. Verwendung einer pharmazeutischen Zubereitung nach einem der Punkte 1 bis 11 in einem Verfahren zur Behandlung und/oder Prophylaxe von Krankheiten, insbesondere von Virusinfektionen vorzugsweise von Infektionen mit HCMV oder einem anderen Vertreter der Gruppe der Herpes viridae.
18. Verwendung einer pharmazeutischen Zubereitung nach einem der Punkte 1 bis 11 zur Herstellung eines Medikaments zur Behandlung und/oder Prophylaxe von Krankheiten, insbesondere von Virusinfektionen vorzugsweise von Infektionen mit HCMV oder einem anderen Vertreter der Gruppe der Herpes viridae.
19. Verfahren zur Bekämpfung von Virusinfektionen vorzugsweise von Infektionen mit HCMV oder einem anderen Vertreter der Gruppe der Herpes viridae in Menschen und Tieren, das die Verabreichung einer pharmazeutischen Zubereitung nach einem der Punkte 1 bis 11 oder einer nach einem der Punkte 12 bis 15 hergestellten pharmazeutischen Zubereitung an einen Menschen oder ein Tier, der (das) ein solche Behandlung benötigt, aufweist.

### Abkürzungsverzeichnis:

- ACN: Acetonitril
- API-ES-pos.: Atmospheric pressure ionization, electrospray, positive (bei MS)
- API-ES-neg.: Atmospheric pressure ionization, electrospray, negative (bei MS)
- ca.: circa
- CI, NH₃: chemische Ionisierung (mit Ammoniak)
- DBU: 1,8-Diazabicyclo[5.4.0]undec-7-en
- DMAP: 4-(Dimethylamino)pyridin
- DMSO: Dimethylsulfoxid
- ESTD: externe Standardisierung
- h: Stunde(n)
- HPLC: Hochdruckflüssigchromatographie (high pressure liquid chromatography)
- konz.: konzentriert(e)
- min.: Minuten
- MS: Massenspektroskopie
- MTBE: Methyl-*tert*-butylether
- NMR: Kernresonanzspektroskopie (nuclear magnetic resonance spectroscopy)
- R_{T}: Retentionszeit (bei HPLC)
- VTS: Vakuumtrockenschrank

### Allgemeine Methoden HPLC:

**Methode 1** (HPLC): Instrument: HP 1050 mit variabler Wellenlängen-Detektion; Säule: Phenomenex-Prodigy ODS (3) 100A, 150 mm × 3 mm, 3 µm; Eluent A: (1,0 g KH₂PO₄ + 1,0 mL H₃PO₄) / l Wasser, Eluent B: Acetonitril; Gradient: 0 min 10% B, 25 min 80% B, 35 min 80% B; Fluss: 0,5 ml/min; Temp.: 45°C; UV-Detektion: 210 nm.

**Methode 2** (HPLC): Instrument: HP 1050 mit variabler Wellenlängen-Detektion; Säule: Chiral AD-H, 250 mm × 4,6 mm, 5 µm; Eluent A: n-Heptan + 0,2 % Diethylamin, Eluent B: Isopropanol + 0,2 % Diethylamin; Gradient: 0 min 12,5 % B, 30 min 12,5 % B; Fluss: 1 ml/min; Temp.: 25°C; UV-Detektion: 250 nm.

**Methode 3** (HPLC): Instrument: HP 1050 mit variabler Wellenlängen-Detektion; Säule: Chiral AD-H, 250 mm × 4,6 mm, 5 µm; Eluent A: n-Heptan + 0,2 % Diethylamin, Eluent B: Isopropanol + 0,2 % Diethylamin; Gradient: 0 min 25 % B, 15 min 25 % B; Fluss: 1 ml/min; Temp.: 30°C; UV-Detektion: 250 nm.

### Beispiele

### A) Herstellung von {8-Fluor-2-[4-(3-methoxyphenyl)piperazin-1-yl]-3-[2-methoxy-5-(trifluormethyl)phenyl]-3,4-dihydrochinazolin-4-yl}essigsäure

### Beispiel 1A

### N-(2-Fluorphenyl)-N'-[2-methoxy-5-(trifluormethyl)phenyl]harnstoff

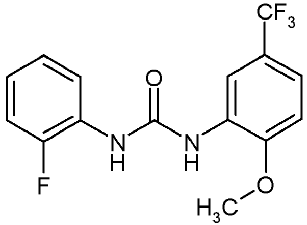

2-Methoxy-5-trifluormethylphenylisocyanat (78 kg) wird bei ca. 35°C aufgeschmolzen und in Acetonitril (insgesamt ca. 270 l) gelöst, dann wird 2-Fluoranilin (39,9 kg) zugegeben und mit Acetonitril (ca. 25 l) nachgespült. Die resultierende klare Lösung wird 4 h unter Rückfluss gerührt und dann auf ca. 75°C abgekühlt. Bei dieser Temperatur wird die Lösung mit Impfkristallen des gewünschten Endprodukts (200 g) angeimpft, für weitere 15 min. gerührt und dann über 3 h auf 0°C abgekühlt. Das erhaltene kristalline Produkt wird durch Zentrifugieren isoliert mit kaltem Acetonitril (zweimal ca. 13 l) gewaschen und bei 45°C im VTS mit Schleppstickstoff getrocknet (ca. 3,5 h). So werden insgesamt 101,5 kg N*-(2-Fluorphenyl)-*N*'-[2-methoxy-5-(trifluormethyl)phenyl]harnstoff* als Feststoff erhalten, entsprechend 85,9 % der Theorie.

¹H NMR (300 MHz, d₆-DMSO): δ = 8,93 (s, 1H), 8,84 (s, 1H), 8,52 (d, ³*J* = 2,3, 2H), 7,55 (d, ²*J* = 7,7, 1H), 7,38-7,26 (m, 3H), 7,22 (d, ²*J* = 8,5, 1H), 4,00 (s, 3H) ppm;
MS (API-ES-pos.): m/z = 409 [(M+H)⁺, 100 %];
HPLC (Methode 1): R_{T} = 22,4 und 30,6 min.

### Beispiel 2A

### Methyl-(2Z)-3-[3-fluor-2-({[2-methoxy-5-(trifluormethyl)phenyl]carbamoyl}amino)-phenyl]acrylat

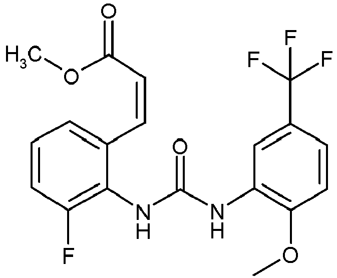

In einem ersten Reaktor werden *N*-(2-Fluorphenyl)-*N*'-[2-methoxy-5-(trifluormethyl)phenyl]harnstoff (51 kg) unter einer Stickstoff-Atmosphäre in Essigsäure (ca. 430 l) gelöst. Zu der entstandenen Lösung wird Methylacrylat (20,1 kg) zugegeben und die entstandene Suspension wird bis zur weiteren Verwendung gerührt. In einem zweiten Reaktor wird Essigsäure (950 l) vorgelegt, Oleum (57 kg) vorsichtig zugegeben und Palladium(II)acetat (7 kg) in der erhaltenen Mischung gelöst. Die in dem ersten Reaktor gebildete Suspension wird nun über ca. 2 h zu der in dem zweiten Reaktor enthaltenen Mischung zugegeben, wobei das Reaktionsgemisch mit einem Gemisch aus 96% Stickstoff und 4% Sauerstoff überströmt wird und die erhaltene Reaktionsmischung wird für ca. 18 h bei Raumtemperatur gerührt. Anschließend wird ein Teil der Essigsäure (ca. 900 l) abdestilliert, zu der verbleibenden Reaktionsmischung wird über ca. 1 h Wasser (ca. 500 l) zugegeben und die erhaltene Suspension wird für 1 h gerührt. Der erhaltene Feststoff wird abfiltriert, einmal mit einem Gemisch aus Essigsäure und Wasser (1 : 1) und zweimal mit Wasser gewaschen und anschließend bei ca. 30 mbar und 50°C getrocknet. So werden insgesamt 44,8 kg *Methyl-(2Z)-3-[3-fluor-2-([[2-methoxy-5-(trifluormethyl)phenyl]carbamoyl}amino)phenyl]acrylat* als Feststoff erhalten, entsprechend 65,0 % der Theorie.

¹H NMR (300 MHz, d₆-DMSO): δ = 9,16 (s, 1H), 8,84 (s, 1H), 8,45 (d, 1,7Hz, 1H), 7,73 (m, 2H), 7,33 (m, 3H), 7,22 (d, 8,6Hz, 1H), 6,70 (d, 16Hz, 1H), 3,99 (s, 3H), 3,71 (s, 3H) ppm; MS (API-ES-pos.): m/z = 429,9 [(M+NH₄)⁺]; 412,9 [(M+H)⁺]
HPLC : R_{T} = 46,4 min.

Instrument: HP 1100 mit variabler Wellenlängen-Detektion; Säule: Phenomenex-Prodigy ODS (3) 100A, 150 mm × 3 mm, 3 µm; Eluent A: (1,36 g KH₂PO₄ + 0,7 ml H₃PO₄) / l Wasser, Eluent B: Acetonitril; Gradient: 0 min 20% B, 40 min 45% B, 50 min 80% B, 65 min 80% B; Fluss: 0,5 ml/min; Temp.: 55°C; UV-Detektion: 210 nm.

### Beispiel 3A

### {8-Fluor-3-[2-methoxy-5-(trifluormethyl)phenyl]-2-oxo-1,2,3,4-tetrahydrochinazolin-4-yl} essigsäuremethylester

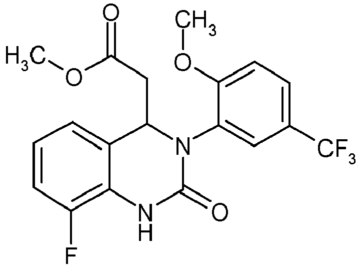

Die Verbindung von Beispiel 2A (75 kg) wird in Aceton (1600 l) suspendiert und DBU (5,7 kg) wird zugegeben. Die resultierende Suspension wird zum Rückfluss erwärmt und 4 h unter Rückfluss gerührt. Die erhaltene Lösung wird auf eine Manteltemperatur von 55°C abgekühlt und über Kieselgur filtriert. Ein Teil des Lösungsmittels wird durch Destillation aus dem Reaktionsgemisch entfernt (ca. 1125 l) und der verbleibende Rückstand wird für 2 h auf 0°C abgekühlt. Der entstandene Feststoff wird durch Zentrifugieren abgetrennt und zweimal mit kaltem Aceton (ca. 15 l) gewaschen und über Nacht bei 45°C unter reduziertem Druck mit Schleppstickstoff bis zur Massenkonstanz getrocknet. So werden insgesamt 58,3 kg *{8-Fluor-3-[2-methoxy-5-(trifluormethyl)phenyl]-2-oxo-1,2,3,4-tetrahydrochinazolin-4-yl}essigsäure-methylester* als Feststoff erhalten, entsprechend 84,1 % der Theorie.
HPLC (Methode 1): R_{T} = 19,4 min.

### Beispiel 4A

### (2S,3,S)-2,3-Bis[(4-methylbenzoyl)oxy]bernsteinsäure-{(4S)-8-Fluor-2-[4-(3-methoxyphenyl)piperazin-1-yl]-3-[2-methoxy-5-(trifluormethyl)phenyl]-3,4-dihydrochinazolin-4-yl}-essigsäuremethylester (1:1-Salz) Chlorierung/Aminierung/Kristallisation

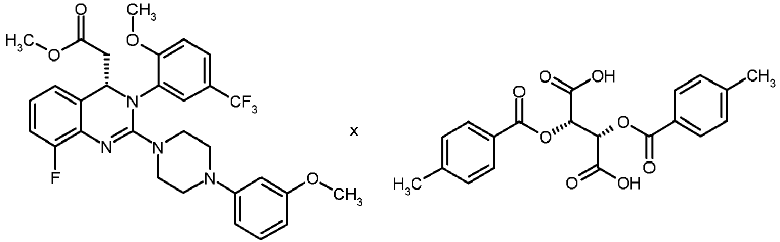

Eine Lösung von {8-Fluor-3-[2-methoxy-5-(trifluormethyl)phenyl]-2-oxo-1,2,3,4-tetrahydrochinazolin-4-yl}essigsäuremethylester (Beispiel 3A, 129,2 kg) in Chlorbenzol (800 l) wird auf Rückfluss erhitzt und azeotrop getrocknet. Phosphoroxychlorid (144 kg) wird zugegeben, und die Reaktionsmischung wird 3 h unter Rückfluss gerührt. Anschließend wird DBU (95 kg) und Chlorbenzol (45 l) zugegeben und für weitere 9 h unter Rückfluss gerührt. Die Reaktionsmischung wird auf Raumtemperatur abgekühlt, durch Zugabe in Wasser hydrolysiert, mit Chlorbenzol (80 l) verdünnt und mit wässriger Ammoniaklösung (25%) neutralisiert. Die Phasen werden getrennt und die organische Phase mit Wasser gewaschen und das Lösungsmittel abdestilliert. Der verbleibende Rückstand wird in Dioxan (170 l) gelöst. 3-Methoxyphenylpiperazin (66 kg), DBU (52 kg) und weitere 90 l Dioxan werden zugegeben und die Reaktionsmischung wird 4 h unter Rückfluss erwärmt. Die Reaktionsmischung wird auf Raumtemperatur abgekühlt, mit Essigester (1300 l) versetzt, 1× mit Wasser, 3 × mit 0.2 N HCl, und 1× mit wässriger NaCl-Lösung gewaschen und das Lösungsmittel wird abdestilliert. Der erhaltene Rückstand wird in Essigester (800 l) gelöst und in eine Lösung aus (2*S*,3*S*)-2,3-Bis[(4-methylbenzoyl)oxy]bernsteinsäure (121 kg) in Essigester (6001) gegeben. Das resultierende Gemisch wird ca. 60 min. bei Raumtemperatur gerührt, dann wird mit (2*S*,3*S*)-2,3-Bis[(4-methylbenzoyl)oxy]-bernsteinsäure-{(4*S*)-8-Fluor-2-[4-(3-methoxyphenyl)-piperazin-1-yl]-3-[2-methoxy-5-(trifluormethyl)-phenyl]-3,4-dihydrochinazolin-4-yl}essigsäuremethylester angeimpft und 3 Tage bei Raumtemperatur gerührt. Anschließend wird auf 0 - 5°C gekühlt und weitere 3 h gerührt. Die Suspension wird abgesaugt und portionsweise mit Ethylacetat nachgewaschen. So werden insgesamt etwa 141 kg (berechnet als trocken) des Salzes als Feststoff erhalten, entsprechend etwa 46,2 % der Theorie über drei Stufen (Chlorierung, Aminierung und Kristallisation) bezogen auf das Racemat.

¹H NMR (300 MHz, d₆-DMSO): δ = 7,90 (d, ²*J* = 7,8, 4H), 7,56 (d, ²*J* = 8,3, 1H), 7,40 (d, ²*J* = 7,8, 4H), 7,28-7,05 (m, 4H), 6,91-6,86 (m, 2H), 6,45 (d, ²*J* = 8,3, 1H), 6,39-6,36 (m, 2H), 5,82 (s, 2H), 4,94 (m, 1H), 4,03 (q, ²*J* = 7,1, 2H), 3,83 (brs, 3H), 3,69 (s, 3H), 3,64 (s, 3H), 3,47-3,36 (m, 8H und Wasser, 2H), 2,98-2,81 (m, 5H), 2,58-2,52 (m, 1H), 2,41 (s, 6H), 1,99 (s, 3H), 1,18 (t, ²*J* = 7,2, 3H) ppm;
HPLC (Methode 1): R_{T} = 16,6 und 18,5 min.

### Beispiel 5A

### (2S,3S)-2,3-Bis[(4-methylbenzoyl)oxy]bernsteinsäure-{(4S)-8-Fluor-2-[4-(3-methoxyphenyl)piperazin-1-yl]-3-[2-methoxy-5-(trifluormethyl)phenyl]-3,4-dihydro-chinazolin-4-yl}essigsäuremethylester (1:1-Salz) / Umkristallisation

(2S,3S)-2,3-Bis[(4-methylbenzoyl)oxy]bernsteinsäure - (S) {(4*S*)-8-Fluor-2-[4-(3-methoxyphenyl)piperazin-1-yl]-3-[2-methoxy-5-(trifluormethyl)phenyl]-3,4-dihydrochin-azolin-4-yl}essigsäuremethylester (1:1-Salz) (141 kg, berechnet als trocken) wird in Ethylacetat (1400 l) suspendiert und durch Erhitzen auf Rückfluss (77°C) gelöst. Die Lösung wird filtriert und langsam auf Raumtemperatur gekühlt. Dabei erfolgt eine spontane Kristallisation. Die Suspension wird 16 h bei RT gerührt, anschließend auf 0-5°C gekühlt und weitere 3 h gerührt. Die Suspension wird abgesaugt und mit kaltem Ethylacetat nachgewaschen. Die Kristalle werden 16 h im Vakuum bei etwa 40 °C getrocknet. So werden insgesamt 131,2 kg des Salzes als Feststoff erhalten, entsprechend 93,0 % der Theorie.
HPLC (Methode 1): R_{T} = 16,9 und 18,8 min.;
HPLC (Methode 3): 99,9 % e.e.

### Beispiel 6A

### (S)-{8-Fluor-2-[4-(3-methoxyphenyl)piperazin-1-yl]-3-(2-methoxy-5-trifluormethylphenyl)-3,4-dihydrochinazolin-4-yl} essigsäure

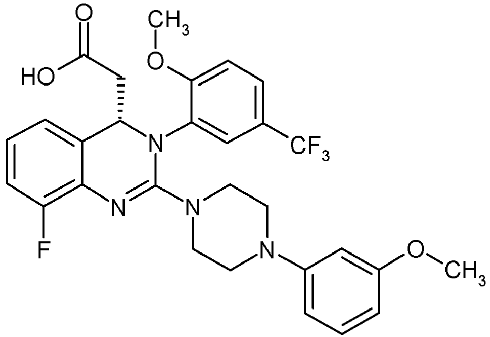

Eine Mischung aus (2*S*,3*S*)-2,3-Bis[(4-methylbenzoyl)oxy]bernsteinsäure-{(4*S*)-8-Fluor-2-[4-(3-methoxyphenyl)piperazin-1-yl]-3-[2-methoxy-5-(trifluormethyl)phenyl]-3,4-di-hydrochinazolin-4-yl}essigsäuremethylester (1:1-Salz) (30,8 kg), Natriumhydrogencarbonat (16.4 kg) und Wasser (315 l) wird mit MTBE (160 l) verrührt. Die Phasen werden getrennt und die organische Phase mit 35 l einer etwa siebenprozentigen wässrigen Natriumhydrogencarbonatlösung behandelt. Die Phasen werden getrennt und die organische Phase wird mit 125 l einer etwa vierprozentigen wässrigen Natriumhydroxidlösung versetzt. Die Reaktionsmischung wird zum Rückfluss erhitzt, das Lösungsmittel schonend bis zum Versiegen destilliert und der Reaktorinhalt anschließend weitere 5 h bei 55 - 60 °C gerührt. Die Reaktionsmischung wird dann bei etwa 22 °C mit MTBE (160 l) und Wasser (65 l) versetzt und verrührt. Die Phasen werden getrennt und die organische Phase mit einer etwa sechsprozentigen wässrigen Natriumchloridlösung (30 l) extrahiert. Die vereinigten wässrigen Phasen werden mit Wasser (25 l) und MTBE (160 l) verrührt und der pH-Wert mit etwa 1N Salzsäure auf etwa 6,5 gestellt. Die organische Phase wird abgetrennt, das Lösungsmittel schonend bis zum Versiegen destilliert und der Rückstand in Aceton (ca. 75 l) gelöst. Es wird ein Lösungsmittelaustausch zu Aceton (6 Destillationsvorgänge mit je ca. 130 1) durchgeführt. Das Zielprodukt wird anschließend durch Zugeben in Wasser ausgefällt, durch Zentrifugieren isoliert und im Vakuumtrockner getrocknet. So werden insgesamt 16,5 kg *(S)-{8-Fluor-2-[4-(3-methoxyphenyl)piperazin-1-yl]-3-(2-methoxy-5-trifluormethylphenyl)-3,4-dihydrochmazolin-4-yl}essigsäure* als amorpher Feststoff erhalten, entsprechend 96,4 % der Theorie.
¹H NMR (300 MHz, d₆-DMSO): δ = 7,53 (d, ²*J* = 8,4, 1H), 7,41 (brs, 1H), 7,22 (d, ²*J* = 8,5, 1H), 7,09-7,01 (m, 2H), 6,86 (m, 2H), 6,45 (dd, ²*J* = 8,2, ³*J* = 1,8, 1H), 6,39-6,34 (m, 2H), 4,87 (t, ²*J* = 7,3, 1H), 3,79 (brs, 3H), 3,68 (s, 3H), 3,50-3,38 (m, 4H), 2,96-2,75 (m, 5H), 2,45-2,40 (m, 1H) ppm;
MS (API-ES-neg.): m/z = 571 [(M-H), 100 %];
HPLC (Methode 1): R_{T} = 15,1 min;
HPLC (Methode 2): 99,8 % e.e.; Pd (ICP): <1 ppm.

### B) Ausführungsbeispiele für pharmazeutische Zubereitungen gemäß der Erfindung

### Beispiel 1

Herstellung einer pharmazeutischen Zubereitung unter Verwendung von Cyclodextrin:
In einem 250 ml-Dreihalskolben werden 30,03 g Hydroxypropyl-(3-Cyclodextrin HP5 (Kleptose HPB, Roquette) mit 68,365 g Wasser für Injektionszwecke gemischt und 6,6 g einer 1 M Natriumhydroxidlösung zu der Mischung zugegeben. Nach Zugabe von 5,005 g der Verbindung von Beispiel 6A wird die Mischung auf 50°C erwärmt und für 24 h gerührt, bis sich eine klare Lösung bildet. Die Lösung wird sterilfiltriert (Porendurchmesser 0,22 µm) und unter aseptischen Bedingungen in sterile 20 ml-Glasbehälter abgefüllt. Die befüllten Glasbehälter werden mit Infusionsstopfen und Bördelkappen verschlossen.

### Beispiel 2

Herstellung einer ersten pharmazeutischen Zubereitung unter Verwendung von Arginin als Hilfsstoff:
Zur Herstellung einer ersten Stammlösung werden 262,38 mg L-Arginin in einen 25 ml-Messkolben eingewogen und anschließend in etwa 22 ml Wasser für Injektionszwecke gelöst. Zu der erhaltenen Argininlösung werden 504,51 mg der Verbindung von Beispiel 6A zugegeben und die Mischung wird für etwa 1 h gerührt, bis eine klare Lösung erhalten wird. Abschließend wird das Volumen mit Wasser für Injektionszwecke aufgefüllt.

Zur Herstellung einer zweiten Stammlösung werden 40,05 mg Natriumdihydrogenphosphat Dihydrat in einen 50 ml-Messkolben eingewogen und in etwa 48 ml Wasser für Injektionszwecke gelöst. Die Mischung wird gerührt, bis eine klare Lösung erhalten wird und das Volumen wird mit Wasser für Injektionszwecke aufgefüllt.

Zur Herstellung einer Lösung mit einer Konzentration von 10 mg pro ml {8-Fluor-2-[4-(3-methoxyphenyl)piperazin-1-yl]-3-[2-methoxy-5-(trifluormethyl)phenyl]-3,4-dihydrochinazolin-4-yl}essigsäure werden in einen 25 ml Messkolben 12,5 ml der ersten Stammlösung mit 10 ml der zweiten Stammlösung gemischt und der pH wird langsam und vorsichtig mit ca. 200 µl 1 M HCl eingestellt. Das Volumen wird dann mit der zweiten Stammlösung aufgefüllt, um eine Mischung mit einem endgültigen pH von 7,9 zu erhalten.

Unter Verwendung dieses Protokolls können Zubereitungen mit unterschiedlichen Konzentrationen von {8-Fluor-2-[4-(3-methoxyphenyl)piperazin-1-yl]-3-[2-methoxy-5-(trifluormethyl)phenyl]-3,4-dihydrochinazolin-4-yl}essigsäure hergestellt werden, wobei lediglich die Menge der eingesetzten ersten Stammlösung variiert werden muss. Ggf. ist hierbei jedoch zu beachten, dass der pH nicht zu stark variiert und insbesondere nicht in den sauren Bereich gerät.

Die so erhaltenen Lösungen werden sterilfiltriert (Porendurchmesser 0,22 µm) und unter aseptischen Bedingungen in sterilisierte Behälter abgefüllt. Die Behälter werden mit Infusionsstopfen und Bördelkappen verschlossen.

### Beispiel 3

Herstellung einer zweiten pharmazeutischen Zubereitung unter Verwendung von Arginin als Hilfsstoff:
Zur Herstellung einer ersten Stammlösung werden 2,1 g L-Arginin in 88,8 g Wasser für Injektionszwecke gelöst. Zu der erhaltenen Argininlösung werden 2 g der Verbindung von Beispiel 6A zugegeben und die Mischung wird für etwa 1 h gerührt, bis eine klare Lösung erhalten wird. Der pH-Wert der erhaltenen Lösung wird durch die tropfenweise Zugabe von 1 M HCl auf 7,8 eingestellt, wobei bei der Zugabe darauf geachtet werden muss, dass diese langsam erfolgt, so dass es nicht zum Ausfallen der Verbindung von Beispiel 6A kommt. Abschließend wird ggf. das Volumen der Lösung auf 100 ml aufgefüllt.

Zur Herstellung einer zweiten Stammlösung werden 3,1 g Natriumdihydrogenphosphat Dihydrat und 8,4 g Glucose in ein geeignetes Gefäß eingewogen und in etwa 74,5 g Wasser für Injektionszwecke gelöst. Die Mischung wird gerührt, bis eine klare Lösung erhalten wird und der pH der erhaltenen Lösung wird mit 1 M NaOH auf einen pH-Wert von 7,8 eingestellt. Abschließend wird ggf. das Volumen der Lösung auf 100 ml aufgefüllt.

Zur Herstellung einer Lösung mit einer Konzentration von 10 mg pro ml {8-Fluor-2-[4-(3-methoxyphenyl)piperazin-1-yl]-3-[2-methoxy-5-(trifluormethyl)phenyl]-3,4-dihydrochinazolin-4-yl}essigsäure werden 50,5 g der ersten Stammlösung mit 53,0 g der zweiten Stammlösung gemischt und für 5 min. gerührt.

Unter Verwendung dieses Protokolls können Zubereitungen mit unterschiedlichen Konzentrationen von {8-Fluor-2-[4-(3-methoxyphenyl)piperazin-1-yl]-3-[2-methoxy-5-(trifluormethyl)phenyl]-3,4-dihydrochinazolin-4-yl}essigsäure hergestellt werden, wobei lediglich die Menge der eingesetzten ersten Stammlösung variiert werden muss. Die so erhaltenen Lösungen werden sterilfiltriert (Porendurchmesser 0,22 µm) und unter aseptischen Bedingungen in sterilisierte Behälter abgefüllt. Die Behälter werden mit Infusionsstopfen und Bördelkappen verschlossen.

### Beispiel 4

Herstellung einer dritten pharmazeutischen Zubereitung unter Verwendung von Arginin als Hilfsstoff:
Zur Herstellung einer ersten Stammlösung werden in einem 100 ml Messkolben 1,05 g L-Arginin und 1 g der Verbindung von Beispiel 6A in etwa 50,0 g Wasser für Injektionszwecke gelöst und die Mischung wird gerührt, bis eine klare Lösung erhalten wird. Der pH-Wert der erhaltenen Lösung wird durch die tropfenweise Zugabe von 0,1 M HCl auf 7,8 eingestellt (ca. 43,5 ml), wobei bei der Zugabe darauf geachtet werden muss, dass diese langsam erfolgt, so dass es nicht zum Ausfallen der Verbindung von Beispiel 6A kommt. Abschließend wird das Volumen der Lösung auf 100 ml aufgefüllt.

Zur Herstellung einer zweiten Stammlösung werden in einem 100 ml Messkolben 1,56 g Natriumdihydrogenphosphat Dihydrat und 4,18 g Glucose in etwa 80,0 g Wasser für Injektionszwecke gelöst. Die Mischung wird gerührt, bis eine klare Lösung erhalten wird und der pH der erhaltenen Lösung wird mit 1 M NaOH auf einen pH-Wert von 7,8 eingestellt (ca. 9,1 ml). Abschließend wird das Volumen der Lösung auf 100 ml aufgefüllt.

Zur Herstellung einer Lösung mit einer Konzentration von 5 mg pro ml {8-Fluor-2-[4-(3-methoxyphenyl)piperazin-1-yl]-3-[2-methoxy-5-(trifluormethyl)phenyl]-3,4-dihydrochinazolin-4-yl}essigsäure werden 50,24 g der ersten Stammlösung mit 51,35 g der zweiten Stammlösung gemischt und für 5 min. gerührt.

Unter Verwendung dieses Protokolls können Zubereitungen mit unterschiedlichen Konzentrationen von {8-Fluor-2-[4-(3-methoxyphenyl)piperazin-1-yl]-3-[2-methoxy-5-(trifluormethyl)phenyl]-3,4-dihydrochinazolin-4-yl}essigsäure hergestellt werden, wobei lediglich die Menge der eingesetzten ersten Stammlösung variiert werden muss.

Die so erhaltenen Lösungen werden sterilfiltriert (Porendurchmesser 0,22 µm) und unter aseptischen Bedingungen in sterilisierte Behälter abgefüllt. Die Behälter werden mit Infusionsstopfen und Bördelkappen verschlossen.

### Beispiel 5

Herstellung einer vierten pharmazeutischen Zubereitung unter Verwendung von Arginin als Hilfsstoff:
Zur Herstellung einer ersten Stammlösung werden in einem 100 ml Messkolben 2,11 g L-Arginin und 2,01 g der Verbindung von Beispiel 6A gemischt und das Volumen wird mit Wasser für Injektionszwecke aufgefüllt. Der pH-Wert dieser ersten Stammlösung betrug 9,8.

Zur Herstellung einer zweiten Stammlösung werden in einem 100 ml Messkolben 3,12 g Natriumdihydrogenphosphat Dihydrat, 8,35 g Glucose und 0,50 g NaCl in etwa 80,0 g Wasser für Injektionszwecke gelöst. Die Mischung wird gerührt, bis eine klare Lösung erhalten wird und der pH der erhaltenen Lösung wird mit 1 M NaOH auf einen pH-Wert von 6,5 eingestellt (ca. 9,7 ml). Abschließend wird das Volumen der Lösung auf 100 ml aufgefüllt.

Zur Herstellung einer Lösung mit einer Konzentration von 10 mg pro ml {8-Fluor-2-[4-(3-methoxyphenyl)piperazin-1-yl]-3-[2-methoxy-5-(trifluormethyl)phenyl]-3,4-dihydrochinazolin-4-yl}essigsäure werden 50,50 g der ersten Stammlösung mit 52,65 g der zweiten Stammlösung gemischt und für 5 min. gerührt.

Unter Verwendung dieses Protokolls können Zubereitungen mit unterschiedlichen Konzentrationen von {8-Fluor-2-[4-(3-methoxyphenyl)piperazin-1-yl]-3-[2-methoxy-5-(trifluormethyl)phenyl]-3,4-dihydrochinazolin-4-yl}essigsäure hergestellt werden, wobei lediglich die Menge der eingesetzten ersten Stammlösung variiert und ggf. der pH-Wert eingestellt werden muss.

Die so erhaltenen Lösungen werden sterilfiltriert (Porendurchmesser 0,22 µm) und unter aseptischen Bedingungen in sterilisierte Behälter abgefüllt. Die Behälter werden mit Infusionsstopfen und Bördelkappen verschlossen.

### Beispiel 6

Herstellung einer pharmazeutischen Zubereitung unter Verwendung von Lysin als Hilfsstoff

Zur Herstellung einer ersten Stammlösung werden 217,24 mg Lysin in einen 25 ml-Messkolben eingewogen und anschließend in 22 ml Wasser für Injektionszwecke gelöst. Zu der so erhaltenen Lösung werden 500,71 mg der Verbindung von Beispiel 6A zugegeben und die Mischung wird für etwa 1 h gerührt, bis eine klare Lösung erhalten wird. Der pH der erhaltenen Lösung wird anschließend mit ca. 460 µl 1 M HCl auf pH 8 gestellt, wobei wiederum darauf geachtet werden muss, dass es nicht zu einer zu starken lokalen Absenkung des pH-Werts und damit verbunden einem Ausfallen der Verbindung von Beispiel 6A kommt. Abschließend wird das Volumen für Injektionszwecke aufgefüllt, um eine erste Stammlösung zu erhalten.

Zur Herstellung einer zweiten Stammlösung werden 242,01 mg Natriumdihydrogenphosphat in einen 50 ml-Messkolben eingewogen und anschließend in etwa 48 ml Wasser für Injektionszwecke gelöst. Die Mischung wird gerührt, bis eine klare Lösung erhalten wird. Der pH der erhaltenen Lösung wird mit ca. 1.825 µl 1 M NaOH auf einen pH-Wert von 8 eingestellt und das Volumen wird dann mit Wasser für Injektionszwecke aufgefüllt.

Zur Herstellung einer Lösung mit einer Konzentration an {8-Fluor-2-[4-(3-methoxyphenyl)piperazin-1-yl]-3-[2-methoxy-5-(trifluormethyl)phenyl]-3,4-di-hydrochinazolin-4-yl}essigsäure von 5 mg pro ml Lösung werden 6,5 ml der ersten Stammlösung in einen 25 ml-Messkolben gefüllt und das Volumen wird mit der zweiten Stammlösung aufgefüllt, um eine Lösung mit einem End-pH von 8 zu erhalten.

Wie bereits z.B. unter Beispiel 2 ausgeführt, können durch Variieren der Menge der ersten Stammlösung auch pharmazeutische Zubereitungen mit anderen Konzentrationen an {8-Fluor-2-[4-(3-methoxyphenyl)piperazin-1-yl]-3-[2-methoxy-5-(trifluormethyl)phenyl]-3,4-dihydrochinazolin-4-yl}essigsäure hergestellt werden.

Die erhaltene Lösung wird sterilfiltriert (Porendurchmesser 0,22 µm) und unter aseptischen Bedingungen in sterilisierte Behälter abgefüllt.

### Beispiel 7

Herstellung einer festen pharmazeutischen Zubereitung, die zur Herstellung einer Infusionslösung rekonstituiert werden kann:
Zur Herstellung einer ersten Stammlösung werden 261,16 mg L-Arginin in einen 25 ml-Messkolben eingewogen und anschließend in 22 ml Wasser für Injektionszwecke gelöst. Zu der erhaltenen Lösung werden 502,45 mg der Verbindung von Beispiel 6A zugegeben und die Mischung wird für etwa 1 h gerührt, bis eine klare Lösung erhalten wird. Der pH dieser Lösung wird mit ca. 660 µl 1 M HCl auf einen Wert von 7,8 eingestellt, wobei wiederum darauf zu achten ist, dass es nicht zu einem Ausfallen der Verbindung von Beispiel 6A kommt und das Volumen wird mit Wasser für Injektionszwecke aufgefüllt.

Zur Herstellung einer zweiten Stammlösung werden 240,05 mg Natriumdihydrogenphosphat in einen 50 ml-Messkolben eingewogen und in etwa 48 ml Wasser für Injektionszwecke gelöst. Die Mischung wird gerührt bis eine klare Lösung erhalten wird. Der pH der erhaltenen Lösung wird mit ca. 1850 µl 1 M NaOH auf einen Wert von 7,8 eingestellt und das Volumen wird mit Wasser für Injektionszwecke aufgefüllt.

Zur Herstellung einer Lösung mit einer Konzentration an {8-Fluor-2-[4-(3-methoxyphenyl)piperazin-1-yl]-3-[2-methoxy-5-(trifluormethyl)phenyl]-3,4-di-hydrochinazolin-4-yl}essigsäure von 10 mg pro ml werden 12,5 ml der ersten Stammlösung in einen 25 ml-Messkolben gegeben und das Volumen wird mit der zweiten Stammlösung aufgefüllt, um eine Lösung mit einem End-pH von 7,8 zu erhalten. Je 1 ml der klaren farblosen Lösung werden in 2 ml Glasbehälter gegeben, die mit einem geeigneten Stopfen versehen werden, und in einem EPSILON 2-4 D Gefriertrockner (Martin Christ GmbH, Deutschland) lyophilisiert, um ein farbloses Pulver zu erhalten, welches durch Zugabe von 1 ml Wasser wieder unproblematisch in eine für eine intravenöse Anwendung geeignete Lösung überführt werden kann.

### Beispiel 8

Herstellung einer festen pharmazeutischen Zubereitung, die zur Herstellung einer Infusionslösung rekonstituiert werden kann:
Zur Herstellung einer ersten Stammlösung werden 210,49 g L-Arginin mit 9665,8 g Wasser für Injektionszwecke vermischt und die Mischung wird gerührt bis eine klare Lösung erhalten wird. Zu der erhaltenen Lösung werden in kleinen Portionen und unter Rühren 199,23 g der Verbindung von Beispiel 6A zugegeben und die Mischung wird gerührt, bis eine klare Lösung erhalten wird, mindestens aber für 30 min.

Zur Herstellung einer zweiten Stammlösung werden 309,19 g Natriumdihydrogenphosphat Dihydrat, 827,47 of Glucose, 49,55 g Natriumchlorid und 7900,0 g Wasser für Injektionszwecke gerührt bis eine klare Lösung erhalten wird. Der pH der erhaltenen Lösung wird mit 1 M NaOH auf einen Wert von 6,55 eingestellt und zu der erhaltenen Lösung werden 1418,29g abzüglich der zur Einstellung des pH-Werts verwendeten Menge an NaOH-Lösung Wasser für Injektionszwecke zugegeben.

Zur Herstellung der gewünschten Lösung wird die zweite Stammlösung langsam und in kleinen Portionen unter leichtem Rühren in die erste Stammlösung gegeben und die erhaltene Lösung wird sterilfiltriert. Je 15 ml der klaren farblosen Lösung werden in entsprechende sterile Glasbehälter gefüllt, die mit einem geeigneten Stopfen versehen werden, und in einem Gefriertrockner lyophilisiert, um ein farbloses Pulver zu erhalten.

Das so erhaltene Lyophilisat kann ohne Probleme z.B. durch Zugaben von 30 ml Wasser für Injektionszwecke wieder zu einer Lösung rekonstituiert werden, die dann ggf. zur Verwendung bei Infusionen noch mal verdünnt wird.

### Beispiel 9

Herstellung einer zweiten pharmazeutischen Zubereitung unter Verwendung von Cyclodextrin:
2,00 g der Verbindung von Beispiel 6A werden eingewogen und mit 30 g einer 0,1 M NaOH-Lösung versetzt und die erhaltene Mischung wird 30 min. gerührt (die Verbindung von Beispiel 6A muss nicht vollständig gelöst sein). Zu der erhaltenen Mischung werden 57,7 g Wasser für Injektionszwecke sowie 15,0 g Hydroxypropyl-β-Cyclodextrin HP5 (Kleptose HPB, Roquette) und 0,31 g NaCl zugegeben und die Mischung wird gerührt bis eine klare Lösung erhalten wird. Die Lösung wird sterilfiltriert (Porendurchmesser 0,22 µm) und unter aseptischen Bedingungen in sterile 20 ml-Glasbehälter abgefüllt. Die befüllten Glasbehälter werden mit Infusionsstopfen und Bördelkappen verschlossen. Die so erhaltenen befüllten Glasbehälter können ggf. hitzesterilisiert werden.

### Beispiel 10

Herstellung einer dritten pharmazeutischen Zubereitung unter Verwendung von Cyclodextrin:
2,00 g der Verbindung von Beispiel 6A werden eingewogen und mit 30 g einer 0,1 M NaOH-Lösung versetzt und die erhaltene Mischung wird 30 min. gerührt (die Verbindung von Beispiel 6A muss nicht vollständig gelöst sein). Zu der erhaltenen Mischung werden 54,8 g Wasser für Injektionszwecke sowie 20,0 g Hydroxypropyl-β-Cyclodextrin HP5 (Kleptose HPB, Roquette) und 0,205 g NaCl zugegeben und die Mischung wird gerührt bis eine klare Lösung erhalten wird. Die Lösung wird sterilfiltriert (Porendurchmesser 0,22 µm) und unter aseptischen Bedingungen in sterile 20 ml-Glasbehälter abgefüllt. Die befüllten Glasbehälter werden mit Infusionsstopfen und Bördelkappen verschlossen. Die so erhaltenen befüllten Glasbehälter können ggf. hitzesterilisiert werden.

### Beispiel 11

Herstellung einer vierten pharmazeutischen Zubereitung unter Verwendung von Cyclodextrin:
0,5 g der Verbindung von Beispiel 6A werden eingewogen und mit 8,75 g einer 0,1 M NaOH-Lösung versetzt und die erhaltene Mischung wird 30 min. gerührt (die Verbindung von Beispiel 6A muss nicht vollständig gelöst sein). Zu der erhaltenen Mischung werden 12,45 g Wasser für Injektionszwecke sowie 5,0 g 2-O-Methyl-β-Cyclodextrin (Crysmeb, Roquette) zugegeben und die Mischung wird gerührt bis eine klare Lösung erhalten wird. Der pH-Wert der Lösung wird mit 1M HCl auf pH 7,5 eingestellt, die Lösung wird sterilfiltriert (Porendurchmesser 0,22 µm) und unter aseptischen Bedingungen in sterile 20 ml-Glasbehälter abgefüllt. Die befüllten Glasbehälter werden mit Infusionsstopfen und Bördelkappen verschlossen. Die so erhaltenen befüllten Glasbehälter können ggf. hitzesterilisiert werden.

### Beispiel 12

Herstellung einer fünften pharmazeutischen Zubereitung unter Verwendung von Cyclodextrin:
0,5 g der Verbindung von Beispiel 6A werden eingewogen und mit 13,125 g einer 0,1 M NaOH-Lösung versetzt und die erhaltene Mischung wird 30 min. gerührt (die Verbindung von Beispiel 6A muss nicht vollständig gelöst sein). Zu der erhaltenen Mischung werden 8,075 g Wasser für Injektionszwecke sowie 5,0 g Sulfoalkylether-β-Cyclodextrin (Captisol, CyDex Pharmaceuticals Inc.) zugegeben und die Mischung wird gerührt bis eine klare Lösung erhalten wird. Der pH-Wert der Lösung wird mit 500µl 1M HCl auf pH 7,5 eingestellt, die Lösung wird sterilfiltriert (Porendurchmesser 0,22 µm) und unter aseptischen Bedingungen in sterile 20 ml-Glasbehälter abgefüllt. Die befüllten Glasbehälter werden mit Infusionsstopfen und Bördelkappen verschlossen. Die so erhaltenen befüllten Glasbehälter können ggf. hitzesterilisiert werden.

Vor der Verabreichung können die beschriebenen Lösung mit einer isotonischen Lösung z.B einer isotonischen Infusionslösung verdünnt werden.

### C) Stabilitätsmessung

Zur Messung der Stabilität wurden die in den Beispielen 1 bis 6 hergestellten Lösungen bei 2 bis 8°C, 25°C, 40°C und für zwei bzw. drei und sechs Wochen gelagert, wobei alle Lösungen eine ausreichende Stabilität zeigten.

Ferner wurde die Stabilität einer aus der Zubereitung von Beispiel 7 hergestellten rekonstituierten Lösung über 24 h bei 2 bis 8°C, 25°C und 40°C getestet, wobei sich die Lösung bei allen Bedingungen über 24 h als stabil erwiesen hat.

### D) Vergleichsversuche für feste pharmazeutische Zubereitungen

Zum Nachweis der vorteilhaften Eigenschaften der in Beispiel 7 erhaltenen festen pharmazeutischen Zubereitung gegenüber anderen festen Zubereitungen wurden die in der Lösung enthaltenen festen Inhaltsstoffe vermischt und anschließend wurden Rekonstitutionsversuche durchgeführt. In keinem der untersuchten Fälle war es hierbei möglich, eine klare Lösung zu erhalten.

### E) Bewertung der physiologischen Wirksamkeit

Die in vitro-Wirkung der erfindungsgemäßen Zubereitungen auf die Replikation des HCMV (humanes Cytomegalovirus) kann in folgendem antiviralen Assay gezeigt werden:

### F) HCMV Fluoreszenzreduktionstest

Die Lösung von Beispiel 8 wird ohne weitere Verdünnung in dem Test eingesetzt. Zum Vergleich wird die Verbidnung von Beispiel 6A als 50 millimolare (mM) Lösungen in Dimethysulfoxid (DMSO) eingesetzt. Als Referenzverbindungen können z.B. Ganciclovir^{®}, Foscarnet^{®} oder Cidofovir^{®} verwendet werden. Einen Tag vor Testansatz werden 1,5 × 10⁴ humane Vorhautfibroblasten (NHDF-Zellen)/well in 200 µl Zellkulturmedium in die Wells B2-G11 von 96-well Platten (schwarz mit durchsichtigem Boden) ausgesät. Die randständigen Wells jeder 96-well Platte werden lediglich mit 200µl Medium befüllt um Randeffekte zu vermeiden. Am Versuchstag wird das Zellkulturmedium der Wells B2 - G11 jeder 96-well Platte abgesaugt und durch 100µl Virussuspension ersetzt (multiplicity of infection (MOI): 0.1-0.2). Bei dem verwendeten Virus handelt es sich um ein rekombinantes HCMV welches eine Expressionskassette für das green fluorescence protein (GFP) in das Virusgenom integriert hat (HCMV AD 169 RV-HG(E. M. Borst, K. Wagner, A. Binz, B. Sodeik, and M. Messerle, 2008, J.Virol. 82:2065-2078.). Nach einer Inkubationszeit von 2h bei 37°C und 5% CO₂ wird das Virus-Inokulat abgesaugt und alle Wells mit Ausnahme der Wells in Spalte 3 werden mit 200µl Zellkulturmedium befüllt. Spalte 2 wird nicht weiter behandelt und dient als Viruskontrolle. Die Wells in Spalte 3 werden jeweils in Doppelbestimmung mit 300µl Zubereitung bzw. Lösung der Testsubstanz (letztere verdünnt in Zellkulturmedium) befüllt. Die Konzentration der jeweiligen antiviralen Substanz in Spalte 3 beträgt ~ die 27fache Konzentration des jeweils erwarteten EC₅₀ Werts. Die Testsubstanz in Spalte 3 wird in 8 Schritten 1:3 über die 96-well Platte verdünnt indem jeweils 100µL einer Spalte in die jeweils rechte Spalte transferiert- und dort mit den vorhandenen 200µl Zellkulturmedium gemischt werden. Auf diese Weise werden drei antivirale Substanzen in Doppelbestimmungen getestet. Die Platten werden 7 Tage bei 37°C / 5% CO₂ inkubiert. Im Anschluss werden alle Wells einer Platte 3 × mit PBS (Phosphate Buffered Saline) gewaschen und mit 50 µl PBS befüllt. Danach wird die GFP-Intensität jedes Wells einer 96-well Platte mittels eines Fluoreszenzlesegerätes (FluoBox; Bayer Technology Services GmbH; Filtereinstellungen: GFP, Ex 480nm, Em 520nm) bestimmt. Aus den so erhaltenen Messwerten kann der EC₅₀ einer anti-HCMV Substanz ermittelt werden:
EC₅₀ (GFP-RA) = Substanzkonzentration in µM welche im Vergleich zur unbehandelten Viruskontrolle die GFP-Fluoreszenz in infizierten Zellen um 50% reduziert.

Repräsentative in-vitro-Wirkdaten für die erfindungsgemäßen Verbindungen sind in Tabelle 1 wiedergegeben:

**Tabelle 1**

| **Virus-Stamm** | **Beispiel 6A EC₅₀ [µM]** | **Beispiel 8 EC₅₀ [µM]** | **Ganciclovir EC₅₀ [µM]** |
|---|---|---|---|
| AD169 RV-HG | 0.0022 ± 0.0002 | 0.0026 ± 0.0005 | 2.5 ± 0.4 |

## Patentansprüche

1. Pharmazeutische Zubereitung, insbesondere zur intravenösen Verabreichung die folgendes aufweist, nämlich:
a) {8-Fluor-2-[4-(3-methoxyphenyl)piperazin-1-yl]-3-[2-methoxy-5-(trifluormethyl)phenyl]-3,4-dihydrochinazolin-4-yl}essigsäure oder ein Salz, ein Solvat oder ein Solvat eines Salzes davon,
b) zumindest einen Hilfsstoff ausgewählt aus den Cyclodextrinen, Lysin und Arginin, und
c) Wasser.

2. Pharmazeutische Zubereitung nach Anspruch 1, die ferner zumindest einen Puffer aufweist, der vorzugsweise ausgewählt ist aus den Phosphat-Puffern, den Tris-Puffern und den Citrat-Puffern.

3. Pharmazeutische Zubereitung nach Anspruch 1 oder 2, die ferner zumindest einen Zucker aufweist, der vorzugsweise ausgewählt ist aus der Gruppe bestehend aus Glucose, Saccharose, Lactose, Maltose, Trehalose, Sorbit und Mannit.

4. Pharmazeutische Zubereitung nach einem der Ansprüche 1 bis 3, **dadurch gekennzeichnet, dass** {8-Fluor-2-[4-(3-methoxyphenyl)piperazin-1-yl]-3-[2-methoxy-5-(trifluormethyl)phenyl]-3,4-dihydrochinazolin-4-yl}essigsäure oder ein Salz, ein Solvat oder ein Solvat eines Salzes davon in einer Menge vorliegt die 1 bis 100 mg, vorzugsweise 2 bis 50 mg, noch bevorzugter 2 bis 25 mg und insbesondere 5 bis 20 mg reiner Wirkstoff pro ml Zubereitung entspricht.

5. Pharmazeutische Zubereitung nach einem der Ansprüche 1 bis 4, **dadurch gekennzeichnet, dass** die Zubereitung einen pH von 7,5 bis 8,5 aufweist.

6. Pharmazeutische Zubereitung nach einem der Ansprüche 1 bis 5, **dadurch gekennzeichnet, dass** der zumindest eine Hilfsstoff in einer Menge von 1 bis 5 Äquivalenten, vorzugsweise von 2 bis 5 Äquivalenten und besonders bevorzugt von 2,5 bis 4,5 Äquivalenten bezogen auf den Gehalt an {8-Fluor-2-[4-(3-methoxyphenyl)piperazin-1-yl]-3-[2-methoxy-5-(trifluormethyl)phenyl]-3,4-dihydrochinazolin-4-yl}essigsäure vorliegt.

7. Pharmazeutische Zubereitung nach einem der Ansprüche 1 bis 6, **dadurch gekennzeichnet, dass** sie bezogen auf 100 ml Folgendes aufweist:
a) 0,2 - 2,0 g, vorzugsweise 0,5 - 1,25 g {8-Fluor-2-[4-(3-methoxyphenyl)piperazin-1-yl]-3-[2-methoxy-5-(trifluormethyl)phenyl]-3,4-dihydrochinazolin-4-yl}essigsäure oder ein Salz, ein Solvat oder ein Solvat eines Salzes davon,
b) 0,25 - 2,5 g, vorzugsweise 0,5 - 1,5 g Arginin,
c) 1,5 - 9,5 g, vorzugsweise 2,0 - 4,75 g Glucose,
d) 0,6 - 4,0 g, vorzugsweise 0,75 - 2,0 g NaH₂PO₄, und
e) Wasser,
wobei die Zubereitung einen pH von 7,5 bis 8,5, vorzugsweise von 7,7 bis 8,0 aufweist.

8. Pharmazeutische Zubereitung nach einem der Ansprüche 1 bis 5, **dadurch gekennzeichnet, dass** der Hilfsstoff ausgewählt ist aus den Cyclodextrinen, vorzugsweise den β-Cyclodextrinen und den modifizierten β-Cyclodextrinen insbesondere den Hydroxyalkyl-β-Cyclodextrinen, den Alkyl-Hydroxyalkyl-β-Cyclodextrinen und den Sulfoalkyl-Cyclodextrinen.

9. Pharmazeutische Zubereitung nach Anspruch 8, **dadurch gekennzeichnet, dass** die Zubereitung bezogen auf den Gehalt an {8-Fluor-2-[4-(3-methoxyphenyl)piperazin-1-yl]-3-[2-methoxy-5-(trifluormethyl)phenyl]-3,4-dihydrochinazolin-4-yl}essigsäure 1 bis 10 Äquivalente, vorzugsweise 2 bis 7 Äquivalente und insbesondere 2,5 bis 5 Äquivalente Cyclodextrin sowie 0 bis 2,0 Äquivalente, vorzugsweise 0,5 bis 1,5 Äquivalent und insbesondere 0,75 bis 0,9 Äquivalente NaOH aufweist.

10. Pharmazeutische Zubereitung nach einem der Ansprüche 1 bis 5 oder nach Anspruch 8 oder 9, **dadurch gekennzeichnet, dass** sie bezogen auf 100 ml Folgendes aufweist:
a) 0,5 - 2,5 g, vorzugsweise 1,0 - 2,0 g {8-Fluor-2-[4-(3-methoxyphenyl)piperazin-1-yl]-3-[2-methoxy-5-(trifluormethyl)phenyl]-3,4-dihydrochinazolin-4-yl}essigsäure oder ein Salz, ein Solvat oder ein Solvat eines Salzes davon,
b) 10,0 - 30,0 g, vorzugsweise 12,5 g - 22,5 g HP-β-Cyclodextrin,
c) 0,0 -350 mg, vorzugsweise 75 - 225 mg, insbesondere 100 - 125 mg NaOH, und
d) Wasser,
wobei die Zubereitung einen pH von 7,5 bis 8,5 aufweist.

11. Feste pharmazeutische Zubereitung, hergestellt durch Lyophilisieren einer pharmazeutischen Zubereitung nach einem der Ansprüche 1 bis 10.

12. Verfahren zur Herstellung einer pharmazeutischen Zubereitung nach einem der Ansprüche 1 bis 7, mit den folgenden Schritten, nämlich:
A) Lösen des zumindest einen Hilfsstoffs in dem Wasser,
B) Zugeben von {8-Fluor-2-[4-(3-methoxyphenyl)piperazin-1-yl]-3-[2-ethoxy-5-(trifluormethyl)phenyl]-3,4-dihydrochinazolin-4-yl}essigsäure oder einem Salz, einem Solvat oder einem Solvat eines Salzes davon zu der in Schritt A) erhaltenen Lösung,
C) ggf. Zugeben des zumindest einen Zuckers und/oder des zumindest einen Puffers,
D) Einstellen des pHs auf den gewünschten Wert, um eine pharmazeutische Zubereitung zu erhalten, und
E) Sterilfiltrieren der in Schritt D) erhaltenen Lösung und Abfüllen in geeignete Behälter.
F) ggf. Endsterilisieren der in Schritt E) erhaltenen Lösung durch Erhitzen.

13. Verfahren zur Herstellung einer pharmazeutischen Zubereitung nach einem der Ansprüche 1 bis 7, mit den folgenden Schritten, nämlich:
I.) Lösen des zumindest einen Hilfsstoffes in einem Teil des Wassers,
II.) Zugeben von {8-Fluor-2-[4-(3-methoxyphenyl)piperazin-1-yl]-3-[2-methoxy-5-(trifluormethyl)phenyl]-3,4-dihydrochinazolin-4-yl}essigsäure oder einem Salz, einem Solvat oder einem Solvat eines Salzes davon zu der in Schritt I.) erhaltenen Lösung,
III.) ggf. Einstellen des pHs der in Schritt II.) erhaltenen Lösung auf den gewünschten Wert, um eine erste Lösung zu erhalten,
IV.) Lösen zumindest eines Zuckers und/oder eines Puffers in einem Teil des Wassers,
V.) ggf. Einstellen des pHs der in Schritt IV.) erhaltenen Lösung auf den gewünschten Wert um eine zweite Lösung zu erhalten,
VI.) Vermischen der ersten und der zweiten Lösung um eine pharmazeutische Zubereitung zu erhalten, und
VII.) Sterilfiltrieren der in Schritt VI.) erhaltenen Lösung und Abfüllen in geeignete Behälter.
VIII.)ggf. Endsterilisieren der in Schritt VII.) erhaltenen Lösung durch Erhitzen.

14. Verfahren zur Herstellung einer pharmazeutischen Zubereitung nach einem der Ansprüche 1 bis 5 oder 8 bis 10, mit den folgenden Schritten, nämlich:
a.) Zugeben von {8-Fluor-2-[4-(3-methoxyphenyl)piperazin-1-yl]-3-[2-methoxy-5-(trifluormethyl)phenyl]-3,4-dihydrochinazolin-4-yl}essigsäure oder einem Salz, einem Solvat oder einem Solvat eines Salzes zu einer wässrigen NaOH-Lösung, vorzugsweise einer wässrigen 0,1 M NaOH-Lösung, zur Herstellung einer Lösung oder Suspension,
b.) Zugeben von Wasser zu der in Schritt a.) erhaltenen Lösung bzw. Suspension,
c.) Zugeben von Cyclodextrin und NaCl zu der in Schritt b.) erhaltenen Lösung bzw. Suspension,
d.) Sterilfiltrieren der in Schritt c.) erhaltenen Lösung und Abfüllen in geeignete Behälter.
e.) ggf. Endsterilisieren der in Schritt d.) erhaltenen Lösung durch Erhitzen.

15. Verfahren zur Herstellung einer festen pharmazeutischen Zubereitung nach Anspruch 11, umfassend die Herstellung einer pharmazeutischen Zubereitung gemäß einem Verfahren nach einem der Ansprüche 12 bis 14 gefolgt von einem Schritt des Lyophilisierens der erhaltenen pharmazeutischen Zubereitung, um eine feste pharmazeutische Zubereitung zu erhalten.

16. Pharmazeutische Zubereitung nach einem der Ansprüche 1 bis 11 zur Verwendung in einem Verfahren zur Behandlung und/oder Prophylaxe von Krankheiten, insbesondere von Virusinfektionen, vorzugsweise von Infektionen mit HCMV oder einem anderen Vertreter der Gruppe der Herpes viridae.
